# FASCICULE DE BREVET EUROPEEN

(11) **EP 0 507 908 B1**
(45) Date de publication et mention de la délivrance du brevet: **02.06.1999**
(21) Numéro de dépôt: 91918401.0
(22) Date de dépôt: 16.10.1991
(51) Int. Cl.: C07K 14/705, C12N 15/12, C07K 16/28, C12Q 1/68

(54) **POLYPEPTIDES AYANT UNE ACTIVITE DE RECEPTEUR DOPAMINERGIQUE HUMAIN, ACIDES NUCLEIQUES CODANT POUR CES POLYPEPTIDES ET UTILISATION DE CES POLYPEPTIDES POUR LE CRIBLAGE DE SUBSTANCES ACTIVES SUR CES POLYPEPTIDES**
POLYPEPTIDE MIT EINER AKTIVITÄT DES MENSCHLICHEN DOPAMINERGISCHEN REZEPTORS, DEREN KODIERENDE NUKLEINSÄURESEQUENZEN UND VERWENDUNG DIESER POLYPEPTIDE
POLYPEPTIDES HAVING HUMAN DOPAMINERGIC RECEPTOR ACTIVITY, NUCLEIC ACIDS CODING FOR SAID POLYPEPTIDES AND USES THEREOF FOR SCREENING ACTIVE SUBSTANCES ON SAID POLYPEPTIDES

(30) Priorité: 06.11.1990 FR 9013731
(43) Date de publication de la demande: 14.10.1992
(73) Titulaire: INSTITUT NATIONAL DE LA SANTE ET DE LA RECHERCHE MEDICALE (INSERM), 75654 Paris Cédex 13 (FR)
(72) Inventeur: GIROS, Bruno, F-92320 Châtillon (FR); SOKOLOFF, Pierre, F-95130 Le Plessis Bouchard (FR); SCHWARTZ, Jean-Charles, F-75014 Paris (FR); MARTRES, Marie-Pascale, F-75015 Paris (FR)
(74) Mandataire: Desaix, Anne
(86) Numéro de dépôt international: FR9100810
(87) Numéro de publication internationale: WO9207937

(56) Documents cités:
- Molecular Pharmacology, vol. 21, 1982, M.W. HAMBLIN et al.: "Phenoxybenzamine treatment differenciates dopaminergic 3H-ligand binding dites in bovine caudate membranes", pages 44-51, voir page 51, colonne 1, lignes 28-47
- Nauyn-Schmiedeberg's Archives of Pharmacology, vol. 315, 1980, P. SOKOLOFF et al.: "Three classes of dopamine receptor (D-2,D-3,D-4) identified by binding studies with 3H-Apomorphine and 3H-Domperidone", pages 89-102, voir page 99, colonne 2; page 100, colonne 1
- Nature, vol. 347, 13 septembre 1990, (Londres, GB), P. SOKOLOFF et al.: "Molecular cloning and characterization of a novel dopamine receptor (D3) as a target for neuroloeptics", pages 146-151, voir l'article en entier

## Description

L'invention a pour objet des polypeptides ayant une activité de récepteur dopaminergique humain et les gènes codant pour ces polypeptides.

L'invention est également relative
- à des vecteurs contenant les gènes codant pour des polypeptides ayant une activité de récepteur dopaminergique,
- à des cellules transformées pour exprimer les gènes susdits sur lesquelles il est possible d'évaluer l'activité d'agents agonistes ou antagonistes desdits récepteurs,
- à des sondes nucléotidiques susceptibles de s'hybrider avec les gènes codant pour les susdits polypeptides, lesquelles sondes seraient susceptibles d'être utilisées pour le diagnostic in vitro d'affections, notamment neurologiques, psychiatriques, cardio-vasculaires ou neuroendocriniennes,
- à des anticorps polyclonaux et monoclonaux dirigés contre les susdits polypeptides et utilisables dans un but analytique pour purifier lesdits polypeptides, de diagnostic in vitro, ou dans un but thérapeutique, notamment dans les affections impliquant les systèmes dopaminergiques,
- à des trousses (ou kits) pour étudier le degré d'affinité de certaines substances pour les susdits polypeptides ,
- à des médicaments destinés plus particulièrement au traitement des affections psychiatriques, neurologiques, cardio-vasculaires ou neuroendocriniennes et contenant des substances actives sur les susdits polypeptides ayant une activité de récepteur dopaminergique, ou contenant des substances actives sur des cellules transfectées par les gènes ou fragments de gènes codant pour lesdits polypeptides à activité de récepteur dopaminergique,
- à des médicaments actifs sur les récepteurs dopaminergiques déjà identifiés mais dont on voudrait diminuer certains effets secondaires attribuables à leur interaction avec lesdits polypeptides.

Il était admis jusqu'alors que les divers effets de la dopamine résultaient de son interaction avec deux types de récepteurs communément désignés sous les noms de récepteurs D-1 et D-2 (Kebabian et Calne, (1979)(1)). Parmi ceux-ci, seule la séquence du récepteur D-2 était connue (Bunzow et al., (1988)(2)) et deux isoformes du récepteur D-2 (parfois désignées D-2A et D-2B ou encore D-2(415) et D-2(444) résultant de l'épissage alternatif de l'ARN messager du gène dudit récepteur avaient été décrites (Giros et al., (1989) (3) ; Dal Toso et al., (1989)(4)).

Le récepteur D-2 appartient à la famille des récepteurs comportant sept domaines transmembranaires, couplés à une protéine G et présentant un certain degré d'homologie avec les récepteurs de la famille des rhodopsines qui sont exprimés par les photorécepteurs de la rétine.

L'existence de récepteurs dopaminergiques distincts des récepteurs D-1 et D-2 avait été suspectée par divers auteurs sur des observations indirectes (voir notamment Schwartz et al., (1984)(5)) mais n'avait jamais été prouvée car lesdits récepteurs n'avaient jamais été isolés, ni identifiés quant à leur structure, ni entièrement définis par leurs propriétés pharmacologiques. En particulier, l'existence d'autorécepteurs régulant la synthèse et/ou la libération de dopamine et/ou encore l'activité des neurones dopaminergiques avait été démontrée mais il était couramment admis qu'ils étaient identiques aux récepteurs D-2 en ce qui concerne leur pharmacologie.

L'existence de polypeptides murins ayant une activité de récepteur dopaminergique, ne s'apparentant ni à l'activité des récepteurs dopaminergiques D-1 ni à celle des récepteurs dopaminergiques D-2 a fait l'objet d'une publication dans Nature (13). Ces polypeptides sont désignés par récepteur dopaminergique D-3 de rat.

L'invention a pour objet de donner accès à de nouveaux polypeptides ayant une activité de récepteur dopaminergique, ne s'apparentant ni à celle des récepteurs dopaminergiques humains D-1, ni à celle des récepteurs dopaminergiques humains D-2.

L'invention a également pour objet de donner accès à de nouvelles séquences d'acides nucléiques permettant un diagnostic spécifique chez l'homme, de certaines pathologies neurologiques ou psychiatriques.

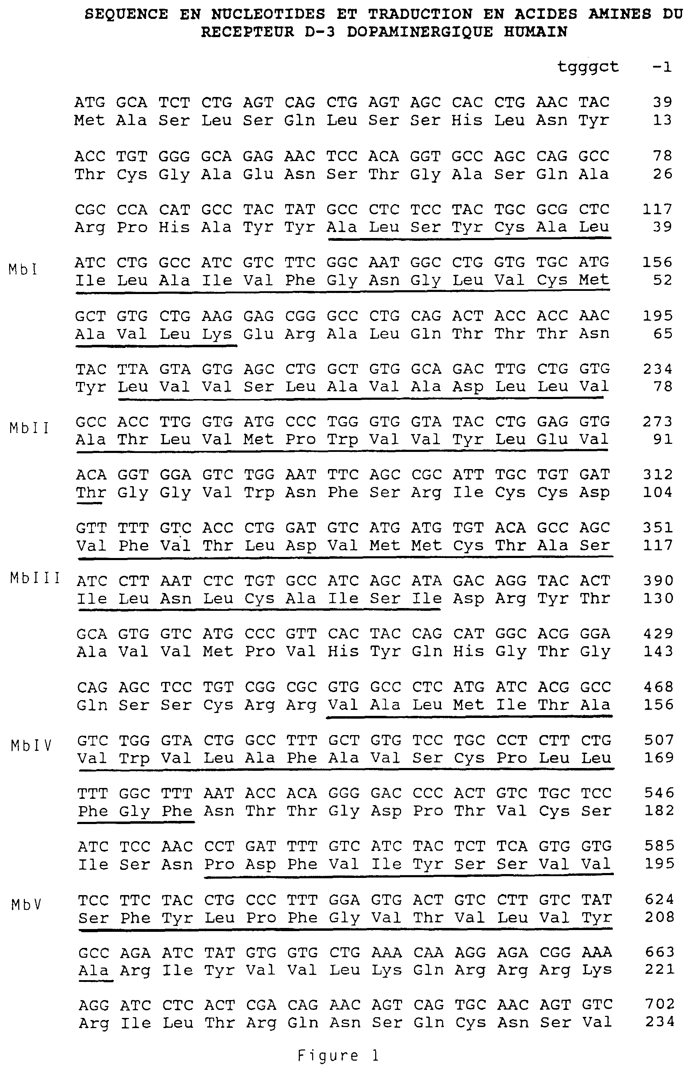

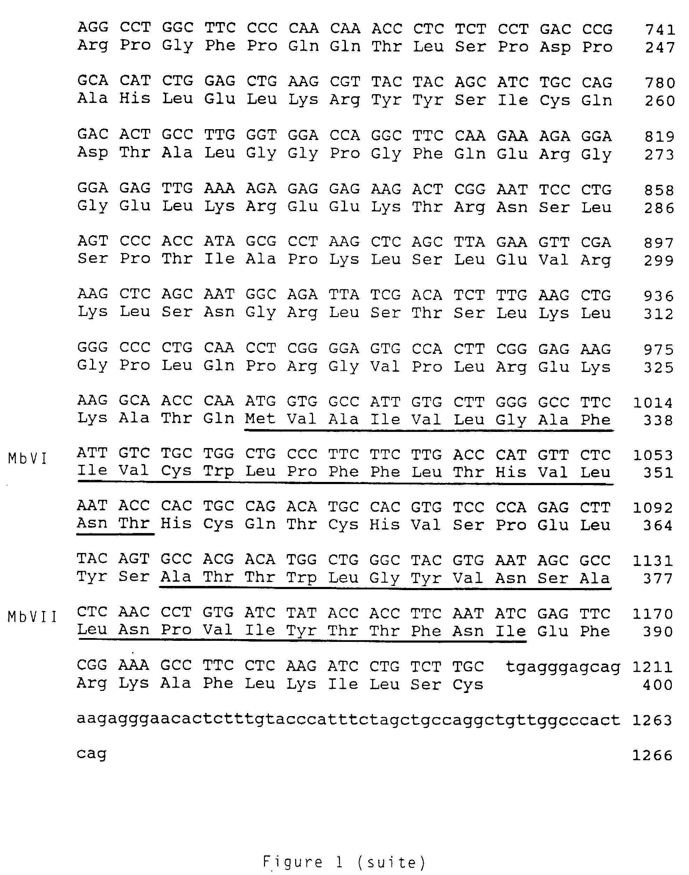

En particulier, le nouveau polypeptide de l'invention ayant une activité de récepteur dopaminergique :
- contient la séquence de 400 acides aminés de la Figure 1 ou un fragment de cette séquence, ce fragment étant tel que

* soit il contient néanmoins les sites contenus dans cette séquence et dont la présence est nécessaire pour que, lorsque ce fragment est présent dans une membrane, il soit capable de lier la dopamine, ses agonistes ou antagonistes de manière spécifique et mesurable, par exemple au moyen d'un ligand radioactif selon la technique décrite dans Sokoloff et al. (1980)(6) et dans Martres et al. (1985)(7), ces sites étant différents des sites contenus dans le récepteur dopaminergique D-3 de rat;
* soit il est susceptible d'être reconnu par des anticorps qui reconnaissent également la susdite séquence de 400 acides aminés, mais ne reconnaissent ni le récepteur dopaminergique D-1, ni le récepteur dopaminergique D-2, ni le récepteur dopaminergique D-3 de rat;
* soit il est susceptible de générer des anticorps qui reconnaissent la susdite séquence de 400 acides aminés mais ne reconnaissent ni le récepteur dopaminergique D-1, ni le récepteur dopaminergique D-2, ni le récepteur dopaminergique D-3 de rat.

Lorsqu'on mentionne "récepteurs dopaminergiques D-3 de rat", il s'agit de celui dont la séquence d'acides aminés et d'acides nucléiques correspondante a été décrite dans l'article de Nature précédemment cité.

La reconnaissance de la susdite séquence de 400 acides aminés par les susdits anticorps - ou du susdit fragment par les susdits anticorps - signifie que la susdite séquence forme un complexe avec l'un des susdits anticorps.

La formation du complexe antigène (c'est-à-dire séquence de 400 acides aminés ou susdit fragment)-anticorps et la détection de l'existence d'un complexe formé peuvent se faire par des techniques classiques (telles que celles utilisant un traceur marqué par des isotopes radioactifs ou une enzyme).

Pour ce qui est de la définition des "fragments contenant les sites" et répondant à la susdite définition, on se reportera ci-après dans la description.

Parmi les fragments ci-dessus définis, on préfère le ou les fragments produits par épissage alternatif de l'ARN messager produit par le gène codant pour la séquence de 400 acides aminés définie ci-dessus.

L'invention concerne également des protéines chimères dans lesquelles le polypeptide tel que défini plus haut ou des parties de celui-ci sont réunis à un enchaînement d'acides aminés hétérologue par rapport à ce polypeptide.

Les polypeptides et protéines chimères de l'invention peuvent être glycosylés et peuvent comporter ou non des ponts disulfure.

Dans la suite de la description, les polypeptides de l'invention seront, pour simplifier, désignés par "récepteurs D-3 humains".

Un récepteur D-3 humain avantageux de l'invention est constitué par l'enchaînement des acides aminés 1 à 400, représentés sur la Figure 1.

Ce récepteur D-3 humain est considéré comme comportant sept régions transmembranaires hydrophobes séparées par des boucles hydrophiles intra et extracellulaires.

Ces régions transmembranaires correspondent aux 7 régions soulignées en regard desquelles MbI correspond à la première région transmembranaire, et ainsi de suite jusqu'à MbVII (septième région transmembranaire).

L'invention concerne également les polypeptides variants qui correspondent aux polypeptides sus-définis comportant certaines mutations localisées, sans que les polypeptides ne perdent les propriétés de récepteur D-3 dopaminergique humain. Parmi ces variants, on peut mentionner ceux qui sont reconnus par des anticorps reconnaissant les régions transmembranaires, ainsi que ceux qui sont reconnus par des anticorps reconnaissant les régions autres que les régions transmembranaires.

Parmi les polypeptides variants de l'invention, on peut citer ceux qui ont l'une au moins des quatre mutations suivantes (les positions des acides aminés susceptibles d'être mutés étant repérées par rapport à la Figure 1) :
Ser en position 9 est remplacé par Gly
Ala en position 79 est remplacé par Val,
Val en position 189 est remplacé par Ile,
Ile en position 397 est remplacé par Thr.

Un polypeptide variant avantageux de l'invention comprend les quatre mutations indiquées ci-dessus.

L'invention concerne également des acides nucléiques qui comprennent ou qui sont constitués par un enchaînement de nucléotides qui, à l'issue de la traduction ou à l'issue de la transcription et de la traduction conduisant à l'un quelconque des récepteurs D-3 humains précédemment définis.

Par "acides nucléiques qui comprennent ou qui sont constitués par un enchaînement de nucléotides qui, à l'issue de la traduction ou à l'issue de la transcription et de la traduction conduisant à", on désigne à la fois :
- les exons,
- les exons flanqués en 5' et/ou en 3' d'introns,
- les exons entrecoupés d'introns,
- les exons flanqués en 5' et/ou en 3' d'introns et entrecoupés d'introns.

L'invention concerne également des acides nucléiques tels que définis ci-dessous, et qui sont caractérisés en ce qu'ils sont constitués par des fragments d'ADN génomique.

Sont exclus de l'invention les acides nucléiques correspondant à la totalité du génome humain, ainsi que ceux correspondant à la totalité du chromosome portant le gène codant pour le susdit récepteur humain D-3.

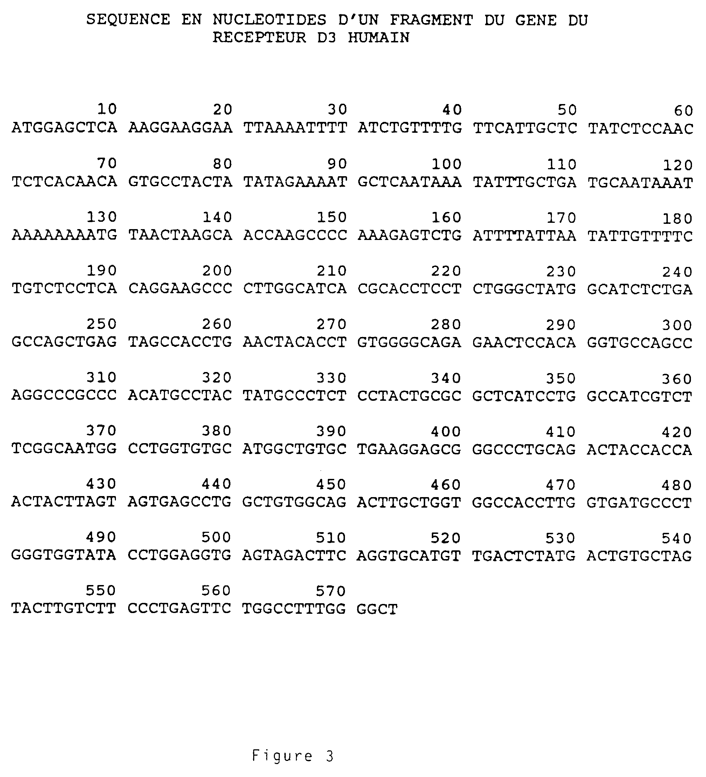

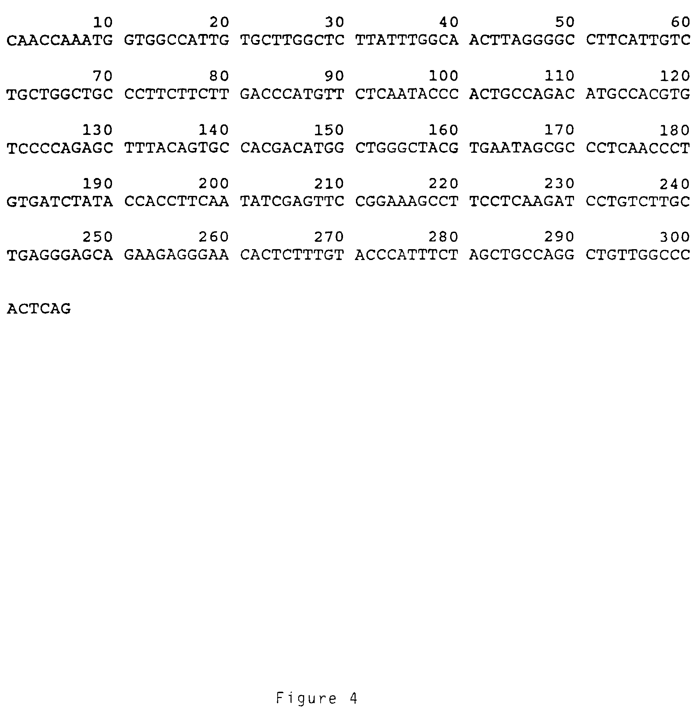

Des acides nucléiques avantageux de l'invention contiennent :
- l'enchaînement d'acides nucléiques représenté sur la Figure 3,
- l'enchaînement d'acides nucléiques représenté sur la Figure 4,
ou sont constitués par l'un des susdits enchaînements.

La séquence d'acides nucléiques délimitée par les nucléotides en position 1 et en position 574, sur la Figure 3, est telle que :
- la séquence s'étendant, sur la Figure 3, de l'extrémité constituée par le nucléotide en position 1 à celle constituée par le nucléotide en position 227, correspond à la partie 5' terminale (non codante) de la séquence d'acides nucléiques codant pour le polypeptide de la Figure 1,
- la séquence s'étendant, sur la Figure 3, de l'extrémité constituée par le nucléotide en position 228 à celle constituée par le nucléotide en position 501, correspond à la séquence (codante) d'acides nucléiques, s'étendant de l'extrémité constituée par le nucléotide en position 1 à celle constituée par le nucléotide en position 274 représentée sur la Figure 1,
- la séquence s'étendant, sur la Figure 3, de l'extrémité constituée par le nucléotide en position 502 à celle constituée par le nucléotide en position 574, correspond à un intron de la séquence d'acides nucléiques codant pour le polypeptide représenté sur la Figure 1.

La séquence d'acides nucléiques délimitée par les nucléotides s'étendant de l'extrémité constituée par le nucléotide en position 1 à celle constituée par le nucléotide en position 306 représentée sur la Figure 4 est telle que :
- la séquence, s'étendant sur la Figure 4, de l'extrémité constituée par le nucléotide en position 1 à celle constituée par le nucléotide en position 46 correspond à un intron de la séquence d'acides nucléiques codant pour le polypeptide représenté sur la Figure 1,
- la séquence, s'étendant sur la Figure 4, de l'extrémité constituée par le nucléotide en position 47 à celle constituée par le nucléotide en position 240 correspond à la séquence (codante) d'acides nucléiques s'étendant de l'extrémité constituée par le nucléotide en position 1007 à celle constituée par le nucléotide en position 1200 représentée sur la Figure 1,
- la séquence s'étendant, sur la Figure 4, de l'extrémité constituée par le nucléotide en position 241 à celle constituée par le nucléotide en position 306 correspond à l'extrémité 3' terminale (non codante) de la séquence d'acides nucléiques codant pour le polypeptide de la Figure 1.

Des acides nucléiques avantageux de l'invention sont ceux contenant ou étant constitués par l'enchaînement d'acides nucléiques de la Figure 3, comportant l'une au moins des mutations suivantes :
A en position 252 est remplacé par G,
A en position 278 est remplacé par G

Des acides nucléiques avantageux de l'invention sont constitués par ceux contenus dans la séquence d'acides nucléiques représentés sur la Figure 3 ou ceux contenant la séquence d'acides nucléiques représentée sur la Figure 3, sous réserve qu'ils hybrident avec la séquence d'acides nucléiques de la Figure 3, dans les conditions indiquées dans les références (3) et (13).

Des acides nucléiques avantageux de l'invention sont constitués par ceux contenus dans la séquence d'acides nucléiques représentés sur la Figure 4 ou ceux contenant la séquence d'acides nucléiques représentée sur la Figure 4, sous réserve qu'ils hybrident avec la séquence d'acides nucléiques de la Figure 4, dans les conditions indiquées dans les références (3) et (13).

Plus particulièrement, l'invention concerne l'acide nucléique qui comprend l'enchaînement de nucléotides représenté sur la Figure 1, s'étendant de l'extrémité constituée par le nucléotide en position -6 à celle constituée par le nucléotide en position 1266.

L'invention concerne particulièrement l'acide nucléique représenté sur la Figure 1 comprenant ou étant constitué par l'enchaînement s'étendant de l'extrémité constituée par le nucléotide en position 1 à celle constituée par le nucléotide en position 1200.

L'acide nucléique sus-défini correspond à la partie codante du gène correspondant au polypeptide représenté sur la Figure 1.

Font également partie de l'invention les acides nucléiques variants par rapport à ceux sus-définis et qui comportent certaines mutations localisées dans la mesure où ces acides nucléiques variants s'hybrident avec les acides nucléiques précédemment définis ou avec les sondes nucléiques définies ci-après dans les conditions d'hybridation définies ci-après dans la description.

Parmi les acides nucléiques variants, on peut citer ceux qui ont l'une au moins des six mutations suivantes (les positions des nucléotides susceptibles d'être mutés étant repérées par rapport à la Figure 1):
A en position 25 est remplacé par G,
A en position 51 est remplacé par G,
C en position 236 est remplacé par T,
G en position 565 est remplacé par A,
T en position 876 est remplacé par C,
T en position 1190 est remplacé par C.

Parmi les acides nucléiques variants, on peut citer celui qui présente aux positions 25, 51, 236, 565, 876 et 1190 l'ensemble des mutations définies ci-dessus.

L'invention vise également les séquences d'acides nucléiques susceptibles d'être obtenues, à partir d'une banque génomique humaine, par hybridation avec l'une des sondes d'hybridation définies ci-après, lesdites séquences d'acides nucléiques pouvant comporter ou être constituées d'introns.

L'invention vise également les acides nucléiques complémentaires de ceux définis ci-dessus, ainsi que les acides nucléiques correspondant à ceux précédemment définis et dans lesquels T est remplacé par U.

Les acides nucléiques de l'invention peuvent être préparés soit par un procédé chimique, soit par d'autres procédés.

Un mode de préparation approprié des acides nucléiques (comportant au maximum 200 nucléotides - ou pb, lorsqu'il s'agit d'acides nucléiques bicaténaires) de l'invention par voie chimique comprend les étapes suivantes :
- la synthèse d'ADN en utilisant la méthode automatisée de β-cyanéthyl phosphoramidite décrite dans Bioorganic Chemistry 4; 274-325, 1986,
- le clonage des ADN ainsi obtenus dans un vecteur plasmidien approprié et la récupération des ADN par hybridation avec une sonde appropriée.

Un mode de préparation, par voie chimique, d'acides nucléiques de longueur supérieure à 200 nucléotides - ou pb (lorsqu'il s'agit d'acides nucléiques bicaténaires) comprend les étapes suivantes:
- l'assemblage d'oligonucléotides synthétisés chimiquement, pourvus à leurs extrémités de sites de restriction différents, dont les séquences sont compatibles avec l'enchaînement en acides aminés du polypeptide naturel selon le principe décrit dans Proc. Nat. Acad. Sci. USA 80; 7461-7465, 1983,
- le clonage des ADN ainsi obtenus dans un vecteur plasmidien approprié et la récupération de l'acide nucléique recherché par hybridation avec une sonde appropriée.

Un autre procédé de préparation des acides nucléiques de l'invention à partir d'ARNm comprend les étapes suivantes :
- préparation d'ARN cellulaire à partir de tout tissu exprimant le récepteur dopaminergique D-3 selon les techniques décrites par Maniatis et al. (1982)(8), et Ausubel F.M. et al. (1989)(9),
- récupération et purification des ARNm par passage des ARN cellulaires totaux par chromatographie avec un oligodT immobilisé,
- synthèse d'un brin d'ADNc à partir des ARNm purifiés d'après la technique décrite dans Gene 25:263, 1983,
- clonage des acides nucléiques ainsi obtenus dans un vecteur plasmidien approprié et récupération de la séquence nucléotidique recherchée en utilisant une sonde d'hybridation appropriée.

Pour préparer les acides nucléiques de l'invention, les sondes d'hybridation oligonucléotidiques synthétisées chimiquement sont les suivantes :
. - celle définie par la séquence d'acides nucléiques représentée sur la Figure 1, s'étendant de l'extrémité constituée par le nucléotide en position -6, à celle constituée par le nucléotide en position 1266,
. - celle définie par la séquence d'acides nucléiques représentée sur la Figure 1, s'étendant de l'extrémité constituée par le nucléotide en position 1, à celle constituée par le nucléotide en position 1200,
. - celle définie par la séquence d'acides nucléiques représentée sur la Figure 1, s'étendant de l'extrémité constituée par le nucléotide en position 720, à celle constituée par le nucléotide en position 831,
. - celle définie par la séquence d'acides nucléiques représentée sur la Figure 3, s'étendant de l'extrémité constituée par le nucléotide en position 1, à celle constituée par le nucléotide en position 574,
. - celle définie par la séquence d'acides nucléiques représentée sur la Figure 4, s'étendant de l'extrémité constituée par le nucléotide en position 1, à celle constituée par le nucléotide en position 306,
ou leur séquence nucléotidique complémentaire;
ces séquences dérivant de celles des Figures 1, 3 et 4, et pouvant être utilisées dans les conditions d'hybridation décrites par Maniatis et al. (1982)(8).

La synthèse du brin d'ADNc et son amplification subséquente in vitro peut également être effectuée en utilisant la méthode PCR (Polymerase Chain Reaction), comme décrit par exemple par Goblet et al. (1989)(10), en utilisant deux amplimères chimiquement synthétisés définis à partir de la séquence de la Figure 1. Des amplimères appropriés sont par exemple : celui défini sur la Figure 1 du nucléotide -6 au nucléotide 18 et celui défini sur la Figure 1 du nucléotide 1226 au nucléotide 1251.

Le fragment d'acides nucléiques amplifié peut être ensuite cloné selon les techniques décrites dans Ausubel F.M. et al. (1989)(11).

L'invention concerne également les vecteurs recombinants, en particulier pour le clonage et/ou l'expression, notamment du type plasmide, cosmide, phage, ou virus, contenant un acide nucléique de l'invention en l'un de ses sites non essentiels pour sa réplication.

Un vecteur approprié de l'invention, contient en l'un de ses sites non essentiels pour sa réplication des éléments nécessaires pour promouvoir l'expression d'un polypeptide selon l'invention, dans un hôte cellulaire et éventuellement un promoteur reconnu par les polymérases de l'hôte cellulaire, en particulier un promoteur inductible et éventuellement une séquence signal et une séquence d'ancrage.

L'invention concerne également un hôte cellulaire transformé par un vecteur recombinant défini précédemment comprenant les éléments de régulation permettant l'expression de la séquence nucléotidique codant pour l'un des polypeptides selon l'invention dans cet hôte.

Par hôte cellulaire on entend tout organisme susceptible d'être maintenu en culture.

L'un des microorganismes utilisés peut être constitué par une bactérie, notamment Escherichia Coli.

Un organisme de choix est constitué par un organisme eucaryote tel que des cellules CHO (Chinese Hamster Ovary) ou COS-7 (fibroblaste de rein de singe vert africain transformé par le virus SV-40).

Mais d'autres organismes peuvent être utilisés tout aussi aisément, naturellement sous réserve que l'on dispose pour chacun d'entre eux des vecteurs, notamment plasmidiques, susceptibles de s'y répliquer et des séquences de nucléotides insérables dans ces vecteurs et capables, lorsqu'elles sont suivies dans ces vecteurs par un insérat codant pour un polypeptide de l'invention, d'assurer l'expression de cet insérat dans les organismes choisis et leur transport dans les membranes de ces hôtes cellulaires.

L'invention concerne également les anticorps dirigés de façon spécifique contre l'un des polypeptides de l'invention, ces anticorps étant tels qu'ils ne reconnaissent ni le récepteur dopaminergique D-1, ni le récepteur dopaminergique D-2. En particulier, ces anticorps reconnaissent les séquences d'acides aminés suivantes :
- celle définie par la séquence d'acides aminés représentée sur la Figure 1 s'étendant de l'extrémité constituée par l'acide aminé en position 243 à celle constituée par l'acide aminé en position 277.

Pour obtenir les anticorps, on peut injecter chez l'animal l'un de susdits polypeptides.

On prépare des anticorps monoclonaux par fusion cellulaire entre des cellules de myélome et des cellules spléniques de souris immunisées, selon les procédés classiques.

Les anticorps de l'invention peuvent être utilisés pour le diagnostic in vitro du caractère tumoral ou non de certaines cellules ainsi que du caractère bénin ou malin de certaines tumeurs, dans la mesure où ces éléments sont corrélés à l'expression pathologique du récepteur D-3 humain.

On a pu constater en effet, que dans certaines tumeurs, notamment tumeurs du poumon, il est possible de détecter l'expression de récepteurs dopaminergiques qui ne devraient pas être normalement présents (Sokoloff et al. (1989) (12)).

Ce procédé de diagnostic in vitro à partir du prélèvement biologique susceptible de contenir le récepteur dopaminergique D-3 humain comprend :
- la mise en contact d'un anticorps de l'invention avec le susdit prélèvement biologique dans des conditions permettant la production éventuelle d'un complexe immunologique formé entre le récepteur dopaminergique D-3 humain ou un dérivé de celui-ci comportant une ou plusieurs mutations localisées et conservant les propriétés de récepteur D-3 dopaminergique humain d'une part, et l'anticorps de l'invention d'autre part, et,
- la détection du susdit complexe immunologique formé.

L'invention concerne également les sondes nucléotidiques synthétiques ou non, s'hybridant avec l'un des acides nucléiques définis ci-dessus ou leurs séquences complémentaires ou leur ARN correspondant, ces sondes étant telles qu'elles s'hybrident ni avec le gène ni avec les ARN messagers des récepteurs D-1 et D-2 dopaminergiques et du récepteur D-3 de rat.

Les sondes de l'invention comportent au minimum 10, avantageusement 15 acides nucléiques et peuvent comporter au maximum la totalité de la séquence nucléotidique représentée sur la Figure 1, sur la Figure 3 ou sur la Figure 4.

Pour les sondes les plus courtes, c'est-à-dire d'environ 10 à environ 100 nucléotides, des conditions d'hybridation appropriées sont les suivantes :
900 mM de NaCl, 90 mM de tri-sodium citrate pH 7,0, 100 µg/ml d'ADN de sperme de saumon, 0,05% de pyrophosphate de sodium, 10 à 25% de formamide déionisée, 0,02% de Ficoll (Pm de 400.000), 0,02% d'albumine de sérum bovine, 0,02% de polyvinylpyrrolidone, pendant 14 à 16 heures à 42°C.

Pour les sondes les plus longues, c'est-à-dire présentant plus d'environ 100 nucléotides, des conditions d'hybridation appropriées sont les suivantes :
600 mM de NaCl, 60 mM de tri-sodium citrate, 20 µg/ml d'ADN de sperme de saumon, 20 µg/ml d'ARNt de levure, 8 mM de Tris-HCl pH 7,4, 40 à 60% de formamide déionisée, 0,02% de Ficoll, 0,02% d'albumine de sérum bovine, 0,02% de polyvinylpyrrolidone, 0,2% de sodium dodécyl sulfate, 10% de sulfate de dextrane.

L'invention concerne en particulier les sondes nucléotidiques définies précédemment.

Les sondes de l'invention peuvent être utilisées comme outils de diagnostic notamment in vitro d'affections neurologiques, psychiatriques et cardio-vasculaires.

Les sondes de l'invention peuvent également servir à détailler l'épissage alternatif de l'ARN messager produit par le gène codant pour la séquence de 400 acides aminés définie ci-dessus, l'existence ou non de cet épissage pouvant être liée à des pathologies neurologiques, psychiatriques, cardio-vasculaires ou neuroendocriniennes.

Plus particulièrement, les sondes de l'invention peuvent être utilisées pour détecter les anomalies génétiques, notamment polymorphismes ou mutations ponctuelles.

En ce qui concerne la détection des polymorphismes du gène codant pour le récepteur dopaminergique D-3 chez un individu, elle peut être réalisée à partir d'un échantillon biologique tel que le sang, prélevé chez l'individu selon un procédé comprenant les étapes suivantes :
- le traitement de l'acide nucléique issu de l'échantillon biologique sus-mentionné réalisé à l'aide d'une enzyme de restriction dans des conditions permettant l'obtention de fragments de restriction issus du clivage dudit acide nucléique au niveau de ces sites de restriction reconnus par ladite enzyme,
- la mise en contact d'une sonde nucléotidique de l'invention, susceptible de s'hybrider avec les fragments sus-mentionnés dans des conditions permettant la production éventuelle de complexes d'hybridation entre ladite sonde et les fragments de restriction sus-mentionnés,
- la détection des susdits complexes d'hybridation,
- la mesure de taille des éventuels fragments de restriction polymorphes engagés dans les complexes d'hybridation sus-mentionnés.

La détection des polymorphismes du gène codant pour le récepteur dopaminergique D-3 chez un individu peut également être réalisée à partir d'un échantillon biologique tel que le sang, prélevé chez l'individu selon un procédé comprenant les étapes suivantes :
- l'amplification préalable possible des quantités de séquences nucléotidiques susceptibles d'être contenues dans un échantillon biologique prélevé sur un patient, au moyen de deux amorces d'ADN,
- le traitement de l'acide nucléique issu de l'échantillon biologique sus-mentionné ou des quantités de séquences nucléotidiques amplifiées comme indiqué ci-dessus, réalisé à l'aide d'une enzyme de restriction dans des conditions permettant l'obtention de fragments de restriction issus du clivage dudit acide nucléique au niveau de ces sites de restriction reconnus par ladite enzyme,
- la mise en contact d'une sonde nucléotidique de l'invention, susceptible de s'hybrider avec les fragments sus-mentionnés dans des conditions permettant la production éventuelle de complexes d'hybridation entre ladite sonde et les fragments de restriction sus-mentionnés,
- la détection des susdits complexes d'hybridation,
- la mesure de taille des éventuels fragments de restriction polymorphes engagés dans les complexes d'hybridation sus-mentionnés.

Comme amorces d'ADN appropriées, on peut utiliser une séquence de 20 bases environ contenues dans la séquence allant du nucléotide 1 au nucléotide 227, représentée sur la Figure 3 et une autre séquence de 20 bases environ contenues dans la séquence allant du nucléotide 502 au nucléotide 574 représentée sur la Figure 4.

Deux amorces préférées sont par exemple :
- celle définie par le nucléotide en position 47 au nucléotide en position 67 de la Figure 3,
- celle définie par le nucléotide en position 489 au nucléotide en position 509 de la Figure 3.

En ce qui concerne la méthode de diagnostic in vitro de détection des mutations ponctuelles de l'acide nucléique ou d'un fragment de l'acide nucléique codant pour le récepteur dopaminergique D-3 chez un individu, elle peut être effectuée selon le procédé comprenant les étapes suivantes :
- la mise en contact d'une sonde nucléotidique de l'invention avec un prélèvement biologique, par exemple du sang, dans des conditions permettant le production éventuelle d'un complexe d'hybridation formé entre la sonde et l'acide nucléique ou un fragment d'acide nucléique sus-mentionné,
- la détection du susdit complexe d'hybridation,
- le séquençage de l'acide nucléique ou du fragment d'acide nucléique intervenant dans le susdit complexe d'hybridation,
- la comparaison de la séquence de l'acide nucléique ou du fragment d'acide nucléique intervenant dans le susdit complexe d'hybridation avec la séquence de l'acide nucléique (ne présentant pas de mutation) ou du fragment de l'acide nucléique (ne présentant pas de mutation) du récepteur dopaminergique D-3 humain.

En ce qui concerne la détection de l'épissage alternatif de l'ARN messager produit par le gène codant pour le récepteur D-3 humain, elle peut se faire par quantification de l'ARN messager contenu dans un échantillon de tissu, en utilisant l'une des sondes de l'invention.

Les sondes de l'invention peuvent également être utilisées pour détecter l'expression pathologique du récepteur dopaminergique D-3, cette expression étant révélée par la présence d'ARN messager du récepteur dopaminergique D-3 dans des cellules où il ne devrait pas être présent.

Cette détection in vitro peut être effectuée à partir d'un échantillon biologique prélevé chez un individu, tel que le sang, selon un procédé qui comprend les étapes suivantes :
- l'amplification préalable possible des quantités de séquence nucléotidique susceptible d'être contenue dans un échantillon biologique prélevé sur un patient, au moyen d'une amorce d'ADN,
- la mise en contact de l'échantillon biologique indiqué ci-dessus avec une sonde nucléotidique, dans des conditions permettant la production d'un complexe d'hybridation formé de ladite sonde et ladite séquence nucléotidique,
- la détection du complexe d'hybridation ci-dessus qui a pu se former.

Cette expression pathologique du récepteur dopaminergique D-3 humain peut se manifester dans le cas de certaines tumeurs, et peut aussi être corrélée au caractère malin ou bénin de certaines tumeurs.

Les polypeptides de l'invention peuvent être préparés par culture dans un milieu approprié d'un hôte cellulaire préalablement transformé par un vecteur recombinant contenant l'un des acides nucléiques définis précédemment et par récupération à partir de la susdite culture du polypeptide produit par ledit hôte cellulaire transformé.

Un autre procédé de préparation des polypeptides de l'invention est caractérisé en ce que, partant de préférence de l'amino acide C-terminal, l'on condense successivement deux à deux les aminoacyles successifs dans l'ordre requis, ou des aminoacyles et des fragments préalablement formés et contenant déjà plusieurs résidus aminoacyles dans l'ordre approprié, ou encore plusieurs fragments préalablement ainsi préparés, étant entendu que l'on aura eu soin de protéger au préalable toutes les fonctions réactives portées par ces aminoacyles ou fragments à l'exception des fonctions amines de l'un et carboxyles de l'autre ou vice versa, qui doivent normalement intervenir dans la formation des liaisons peptidiques, notamment après activation de la fonction carboxyle, selon les méthodes connues dans la synthèse des peptides et ainsi de suite, de proche en proche, jusqu'à l'acide aminé N-terminal.

S'agissant de réaliser l'expression des récepteurs dopaminergiques D-3 humains dans une bactérie, telle que E. coli ou dans une cellule eucaryote telle qu'une cellule CHO, on effectue les étapes suivantes :
- la transformation d'un hôte cellulaire compétent avec un vecteur, notamment un plasmide ou un phage, dans lequel a auparavant été insérée une séquence de nucléotides codant pour le récepteur D-3 humain (insérat), sous le contrôle d'éléments de régulation, notamment d'un promoteur reconnu par les polymérases de l'hôte cellulaire et permettant l'expression dans l'hôte cellulaire utilisé de ladite séquence de nucléotides,
- la culture de l'hôte cellulaire transformé dans des conditions permettant l'expression dudit insérat, et le transport du récepteur D-3 humain exprimé vers la membrane de façon telle que les séquences transmembranaires du récepteur D-3 humain soient exposées à la surface de l'hôte cellulaire transformé.

S'agissant de l'expression en cellules eucaryotes, les éléments de régulation peuvent comporter le promoteur endogène des récepteurs dopaminergiques ou des promoteurs viraux tels que ceux des virus SV40 ou du virus du sarcome de Rous (RSV).

S'agissant de l'expression dans E. coli, les éléments de régulation peuvent comporter le promoteur de l'opéron lactose ou de l'opéron tryptophane.

L'invention concerne également un procédé de détection de la capacité d'une molécule dopaminergique à se comporter comme ligand vis-à-vis d'un polypeptide de l'invention, lequel procédé comprend :
- la mise en contact de la molécule dopaminergique avec un hôte cellulaire préalablement transformé par un vecteur lui-même modifié par un insérat codant pour le susdit polypeptide, cet hôte portant à sa surface un ou plusieurs sites spécifiques de ce polypeptide, le cas échéant après induction de l'expression de cet insérat, cette mise en contact étant effectuée dans des conditions permettant la formation d'une liaison entre l'un au moins de ces sites spécifiques et ladite molécule dès lors qu'elle s'avérerait effectivement posséder une affinité pour ce polypeptide,
- la détection de la formation éventuelle d'un complexe du type ligand-polypeptide.

L'invention concerne également un procédé pour l'étude de l'affinité d'un polypeptide de l'invention pour un ou plusieurs ligands déterminés, lequel procédé comprend :
- la transformation d'un hôte cellulaire compétent avec un vecteur, notamment un plasmide ou un phage, dans lequel avait auparavant été insérée une séquence de nucléotides codant pour le récepteur D-3 humain (insérat), sous le contrôle d'éléments de régulation, notamment d'un promoteur reconnu par les polymérases de l'hôte cellulaire et permettant l'expression dans l'hôte cellulaire utilisé de ladite séquence de nucléotides,
- la culture de l'hôte cellulaire transformé dans des conditions permettant l'expression dudit insérat, et le transport du récepteur D-3 humain exprimé vers la membrane de façon telle que les séquences transmembranaires du récepteur D-3 humain soient exposées à la surface de l'hôte cellulaire transformé,
- la mise en contact de cet hôte cellulaire avec ces ligands déterminés,
- la détection d'une liaison spécifique entre ledit hôte cellulaire transformé et lesdits ligands déterminés.

Le procédé décrit ci-dessus permet également l'identification des "fragments contenant les sites" dont question précédemment et qui ne comportent qu'une partie de la séquence de 400 acides aminés de la Figure 1.

Cette identification consiste à utiliser des insérats de taille plus réduite que l'acide nucléique codant pour la susdite séquence, à mettre en oeuvre les étapes relatives à l'expression, au transport du produit d'expression et à l'exposition rappelées ci-dessus. Lorsqu'on obtient l'expression, le transport du produit d'expression et son exposition sur la membrane, ainsi que la réaction avec les ligands telle que précédemment définie, on peut déterminer ainsi les fragments contenant les sites essentiels. Par conséquent, l'absence de réaction avec les ligands, telle que précédemment définie, en utilisant des fragments de taille plus réduite que la séquence complète, tendrait à montrer qu'on a éliminé certains sites essentiels.

D'autres caractéristiques et avantages de l'invention apparaissent dans la suite de la description et des exemples, notamment en rapport avec les dessins et tableaux dans lesquels :
- La Figure 1 représente la séquence d'acides nucléiques du récepteur dopaminergique D-3 humain, et la séquence d'acides aminés correspondante.

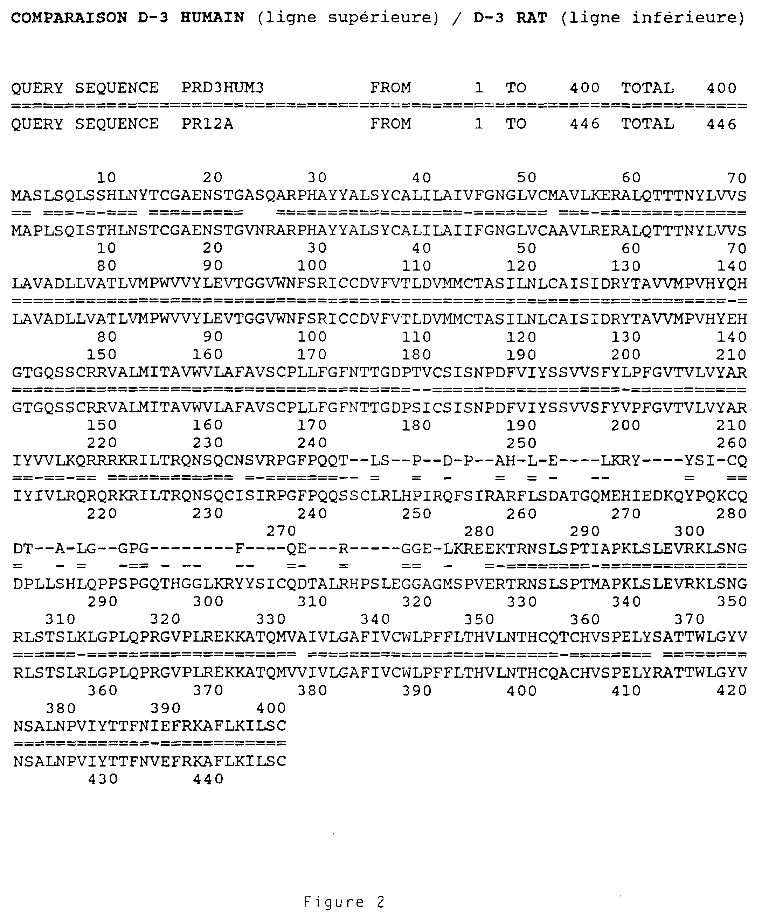

- La Figure 2 représente les séquences d'acides aminés du récepteur dopaminergique D-3 (deuxième ligne) de rat, alignée avec celle du récepteur dopaminergique D-3 humain (première ligne). Dans les séquences du récepteur dopaminergique D-3 de rat, les résidus d'amino acides identiques et en position analogue à ceux du récepteur dopaminergique D-3 humain sont marqués par des doubles traits (=).

Pour mettre en évidence les homologies, on a effectué des délétions dans les deux séquences, et ces délétions sont représentées par les espaces pointillés (-) dans la séquence du récepteur D-3 de rat.

Les traits simples entre les lignes correspondent respectivement au récepteur D-3 humain et au récepteur D-3 de rat correspondant au fait qu'il n'y a pas homologie.

Dans la région N-terminale extra-cellulaire du récepteur D-3, les séquences consensus pour les sites de glycosylation liés à l'asparagine (NXS/T) sont en positions 12 et 19 (Figure 2).

Le pourcentage d'homologie existant entre le récepteur D-3 de rat et le récepteur D-3 humain est de 78,7%.
- La Figure 3 correspond à une partie du gène du récepteur D-3 humain du côté 5'-terminal de la partie codante; la séquence s'étendant, sur la Figure 3, de l'extrémité constituée par le nucléotide en position 228 à celle constituée par le nucléotide en position 501, correspond à la séquence (codante) d'acides nucléiques, s'étendant de l'extrémité constituée par le nucléotide en position 1 à celle constituée par le nucléotide en position 274 représentée sur la Figure 1.
- La Figure 4 correspond à une partie du gène du récepteur D-3 humain du côté 3'-terminal de la partie codante; la séquence s'étendant, sur la Figure 4, de l'extrémité constituée par le nucléotide en position 47 à celle constituée par le nucléotide en position 240 correspond à la séquence (codante) d'acides nucléiques s'étendant de l'extrémité constituée par le nucléotide en position 1007 à celle constituée par le nucléotide en position 1200 représentée sur la Figure 1.

### EXEMPLE 1 : Détermination de la séquence en nucléotides et traduction en amino-acides du récepteur dopaminergique D-3 humain :

### Méthode

Une banque génomique humaine Sau 3A-partielle dans EMBL 3 (Clontech) a été criblée à l'aide de deux ADNc radiomarqués au ³²P, obtenus par coupure d'ADNc codant pour le récepteur D-3 de rat; l'un des ADNc est obtenu à partir du récepteur D-3 de rat coupé par ClaI-PstI, ce qui donne la sonde 1 (partie N-terminale + région transmembranaire MbI + région transmembranaire MbII) et l'autre ADNc est obtenu à partir du récepteur D-3 de rat coupé par BstXI-EcoRI, ce qui donne la sonde 2 (partie C-terminale + région transmembranaire MbVI + région transmembranaire MbVII) (Sokoloff et al., 1990).

Les phages de la banque génomique (940.000 clones) sont transférés sur des membranes en nitrocellulose (BAS-85, Schleicher et Schuell) qui sont préhybridées (40% formamide, 4xSSC, 1 x Dehnardt's, 8 mM Tris-HCl pH 7.4, 20 µg/ml ARNt de levure, 20 µg/ml de sperme de saumon, 0,1% SDS), 4 heures à 42°C et hybridées (même solution + sulfate de dextran 10%), 14 heures à 42°C en présence de 700.000 cpm/ml de sonde 1 ou de sonde 2 définie ci-dessus (activité spécifique 10⁹ cpm/µg ADN).

Puis, les membranes sont lavées deux fois 15 minutes à 42°C (2 x SSC; 0,1% SDS) et deux fois 15 minutes à 42°C et 50°C respectivement (0,2 x SSC; 0,1% SDS). Puis, les membranes sont apposées à des films radiographiques (XAR 5, Kodak) à -70°C en présence d'écrans amplificateurs. Les films radiographiques sont révélés après 5 heures d'exposition.

Un clone positif est obtenu avec la sonde 2 (extrémité 3'), qui contient un fragment EcoRI réactif de 4,8 kb qui est subcloné en M13 et séquencé.

Cinq clones positifs sont obtenus avec la sonde 1 (extrémité 5'), qui contiennent des fragments réactifs SacI de 1,2 kb, et EcoRI de 2,5 kb, qui sont subclonés en M13 et séquencés.

La séquence d'ADN génomique permet de constater la présence de phases ouvertes de lectures présentant une forte homologie avec la séquence du récepteur D-3 de rat.

Des amorces utilisables pour l'amplification PCR sont synthétisées :
en 3': A : TTG AAT TCC TGG CAG CTA GAA ATG GGT ACA A en 5': B : CGA ATT C AT G GC ATC TCT GAG CCA GCT GAG TA en 3':

C : AAG AAT TCA GAT CTT CTG CTC CCT CAG CAA G

Un simple brin d'ADNc est synthétisé à partir d'ARNm de corps mamillaires humains et de transcriptase reverse (20 U, Boehringer) avec l'amorce B(375 mM).

Cette matrice est amplifiée par la polymérase de Thermophylus Aquaticus (Cetus, 2,5 U) à l'aide des amorces B et A (75 mM chaque) pendant 30 cycles (92°C, 56°C, 72°C, 1 minute chaque).

Les produits de la réaction sont séparés par électrophorèse sur un gel d'agarose de 1%, et une bande du gel (entre 1 et 1,4 kb) est découpée, l'ADN extrait (gene-clean, BIO 101) et réamplifié entre les amorces B et C. L'ADN (1,2 kb) alors visible par coloration au bromure d'éthidium sur un gel d'agarose est extrait à nouveau, phosphorylé par la polynucléotide kinase (10 U, Boehringer), et subcloné dans pGEM 4Z (Promega) coupé par SmaI.

Un fragment entier est extrait du plasmide par KpnI et BglII et subcloné dans M13 pour séquençage.

La séquence donne une phase ouverte de lecture de 400 acides aminés avec 79% d'homologie globale avec le récepteur D-3 de rat, et 93% si l'on ne considère pas la troisième boucle intracytoplasmique.

### EXEMPLE II : Expression du récepteur D-3 dans des cellules CHO transfectées :

On a mesuré l'inhibition de la liaison d'iodosulpride-¹²⁵I par des agonistes et des antagonistes de la dopamine.

### METHODE

Les vecteurs d'expression dérivent du plasmide pSV D-2 dont la construction est indiquée dans l'article de Nature mentionné ci-dessus.

Le vecteur, pour exprimer le récepteur D-3 humain, est obtenu en remplaçant le fragment de restriction HindIII-BglII de PSV D-2 par un fragment de restriction BglII-KpnI du plasmide pGEM 4Z en utilisant un adaptateur d'oligonucléotide HindIII-KpnI, (AGCTGTAC) conduisant au plasmide pSV D-3 humain.

Ces constructions sont cultivées et purifiées sur gradient de chlorure de cesium (Sambrook J. et al., Molecular cloning - a laboratory manual. C. Nolan, ed., Cold Spring Harbor Laboratory Press, 1989) et transfectées dans des cellules d'ovaire de hamster chinois (CHO-K1) déficientes en dihydrofolate réductase en utilisant la Lipofectine® (Bethesda Research Laboratory, Gaithersburgh, USA). Les transfectants stables sont sélectionnés dans un milieu de culture (Dubelcco's Modified Eagle medium) sans hypoxanthine et sans thymidine.

L'expression du récepteur D-3 est mise en évidence par la liaison de l'iodosulpride-¹²⁵I à des membranes de cellules transfectées selon la technique de Martres et coll. 1985 (7). Les expériences de liaison sont effectuées (11) dans un tampon Tris-HCl 50mM (volume total 400µl) contenant NaCl 120mM, KCl 5mM, CaCl₂ 2 mM, MgCl₂ 2mM, 0,1% d'acide ascorbique, 8-hydroxy quinoléine 10µM, 0,1% d'albumine de sérum bovin et iodosulpride-¹²⁵I 0,1 à 0,2nM.

### RESULTATS

Le récepteur D-3 humain a été exprimé dans des cellules CHO, qui normalement n'ont pas de sites de liaison d'iodosulpride-¹²⁵I.

Dans des clones transfectés en permanence de cellules CHO, la valeur de K_{D} est de 1,1 nM, et la valeur B max (Bmax représentant la concentration maximale en récepteur D-3) est d'environ 0,6 pmol/mg de protéine de membrane.

Les résultats relatifs à la pharmacologie du récepteur D-3 humain exprimé dans des cellules CHO sont indiqués ci-après :

| Agents | Valeur K_{I} (nM) |
|---|---|
| - Agonistes : | |
| . dopamine | 39,6 ± 4,2 |
| . dopamine + Gpp(NH)p | 43,5 ± 3,9 |
| . quinpirole | 10,3 ± 1,0 |

| - Antagonistes : | |
|---|---|
| . domperidone | 4,33 ± 0,40 |
| . haloperidol | 4,25 ± 0,61 |
| . amisulpride | 2,20 ± 0,20 |
| . pimozide | 0,67 ± 0,06 |
| . (+)UH 232 | 22,05 ± 1,70 |
| . iodosulpride | 1,10 ± 0,10 |

### Exemple III - Modèle d'étude de la fonction du récepteur D-3 humain:

La cellule CHO transfectée avec le récepteur D-3 humain ne permet pas de mesurer une réponse fonctionnelle à la stimulation de ce récepteur. Dans cette cellule, il n'a pu être mis en évidence ni activation ni inhibition des voies de transduction connues (acénylate cyclase, phospholipase C et phospholipase A₂). Ceci a été attribué au fait que la cellule CHO n'exprime pas la protéine G transductrice spécifique du couplage avec le récepteur D-3.

Par exemple, on sait que la cellule CHO n'exprime pas la protéine G₀, alors qu'elle exprime d'autres protéines appartenant à la même famille de protéines G.

Cette hypothèse s'est révélée exacte puisqu'une cellule CHO transfectée à la fois avec le récepteur D-3 humain et la sous-unité α₀ de la protéine G₀ (protéine α₀) a permis de mesurer une réponse fonctionnelle.

### METHODES

Un acide nucléique codant la protéine α₀ de rat a été obtenu par amplification PCR avec des amorces dérivées de la séquence publiée de la sous unité α₀. La séquence de la sous unité α₀ a été publiée par Jones et Reed (Proc. Natl. Acad. Sci. USA 1987, 262: 14241-14249).

Les amorces uitlisées sont les suivantes :

en 5': TTA.TCT.AGA.AGG.GGC.CAC.CAT.GGG.ATG.TA

en 3': TAC.GAG.CTC.AAG.AAG.TCA.GTA.CAA.GCC.AC

L'acide nucléique obtenu a été digéré par les enzymes de restriction SacI et XbaI (sites contenus dans les amorces) et subcloné dans un vecteur d'expression pEUK-C (Clontech Laboratories, Inc. Palo Alto, Etats-Unis). Ce plasmide a été préparé et transfecté comme décrit dans l'exemple II en même temps qu'un plasmide pUT 523 (Cayla, Toulouse, France) contenant un gène de résistance à la phléomycine. Les transfectants ont été sélectionnés dans le même milieu de culture que celui de l'exemple précédent contenant de la phléomycine.

Sur ces cellules, la variation de la concentration de calcium intracellulaire a été enregistrée selon la technique décrite par Malgaroli et coll. (J. Cell. Biol. 1987, 105: 2145-2155) et Vallar et coll. (J. Biol. Chem. 1988, 263: 10127-10134).

### RESULTATS

Sur la cellule CHO exprimant à la fois le récepteur D-3 humain et la protéine α₀, on a constaté que la dopamine augmente la concentration de calcium intracellulaire avec une concentration efficace 50 de l'ordre de 1 nM. Pour mémoire, la concentration efficace 50 correspond à la valeur de la concentration de dopamine avec laquelle on obtient la moitié de l'augmentation maximale de la concentration de calcium intracellulaire . La réponse à la dopamine 100 nM est complètement antagonisée par un antagoniste vis à vis du récepteur dopaminergique D-3 humain tel que le (+)UH 232 à la concentration de 1 µM.

Ce modèle fonctionnel permet de détecter le pouvoir agoniste ou antagoniste vis à vis du récepteur dopaminergique D-3 humain des molécules et d'évaluer les concentrations efficaces 50 des agonistes.

Pour déterminer le pouvoir agoniste ou antagoniste d'une molécule vis à vis du récepteur D-3, on peut mettre en présence une cellule hôte, telle qu'une cellule CHO, transfectée à la fois par le récepteur dopaminergique D-3 humain et la sous-unité α₀ de la protéine G₀, avec une molécule pour laquelle on veut déterminer le pouvoir agoniste ou antagoniste,
- s'il y a augmentation de la concentration de calcium intracellulaire par rapport à la concentration de calcium intracellulaire en l'absence de ladite molécule, on en déduit que la molécule est un agoniste vis à vis de D-3;
- s'il n'y a pas modification de la concentration de calcium intracellulaire, on met la susdite cellule hôte en présence de dopamine et de la susdite molécule, et s'il y a diminution de la concentration de calcium intracellulaire par rapport à celle obtenue en présence de dopamine, on en déduit que la molécule est un antagoniste vis-à-vis de D-3.

Pour déterminer la concentration efficace 50 d'une molécule dont on a défini la nature agoniste, on détermine la concentration de cette molécule qui produit la moitié de l'augmentation maximale de la concentration de calcium intracellulaire.

### BIBLIOGRAPHIE

1. Kekabian J.W. et Calne D.B., "Multiple receptors for dopamine". Nature, 1979, 277: 93-96.
2. Bunzow J.R., Van Tol H.H.M., Grandy D.K., Albert P., Salon J., Christie Mc D., Machida C.A., Neve K.A. et Civelli O. "Cloning and expression of a D-2 dopamine receptor cDNA". Nature, 1988, 336: 783-787.
3. Giros B., Sokoloff P., Martres M.P., Riou J.F., Emorine L.J. et Schwartz J.C. "Alternative splicing directs the expression of two D-2 dopamine receptor isoforms". Nature, 1989, 342: 923-926.
4. Dal Toso R., Sommer B., Ewert M., Herb A., Pritchett D.B., Bach A., Shivers B.D. et Seeburg P.H. "The dopamine D-2 receptor : two molecular forms generated by alternative splicing". The EMBO Journal, 1989, 8: 4025-4034.
5. Schwartz J.C., Delandre M., Martres M.P., Sokoloff P., Protais P., Vasse M., Costentin J., Laibe P., Wermuth C.G., Gulat C. et Lafitte A. "Biochemical and behavioral identification of discriminant benzamide derivatives : new tools to differentiate subclasses of dopamine receptors". Catecholamines: neuropharmacology and central nervous system. Theoretical aspects. Alan R. Liss, Inc. N.Y. 1984, pp. 59-72.
6. Sokoloff P., Martres M.P. et Schwartz J.C. "Three classes of dopamine receptor (D-2, D-3, D-4) identified by binding studies with ³H-apomorphine and ³H-domperidone". Naunyn-Schmiedeberg's Arch. Pharmacol. 1980, 315: 89-102.
7. Martres M.P., Bouthenet M.L., Sales N., Sokoloff P. et Schwartz J.C. "Widespread distribution of brain dopamine receptors evidenced with ¹²⁵I-iodosulpride, a highly selective ligand". Science, 1985, 228: 752-755.
8. Maniatis et al., "Molecular cloning", Cold Spring Harbor Laboratory, 1982.
9. Ausubel F.M., Brent R., Kingston R.E., Moore D.D., Smith J.A., Seidman J.G. et Struhl K. (1989) Current Protocols in Molecular Biology, chapître 4, Green Publishing Associates et Wiley-Interscience, New York.
10. Goblet et al. Nucleic Acid Research, 17, 2144, 1989 "One step amplification of transcripts in total RNA using Polymerase Chain Reaction".
11. Ausubel F.M., Brent R., Kingston R.E., Moore D.D., Smith J.A., Seidman J.G. et Struhl K. (1989) Current Protocols in Molecular Biology, chapître 3, Green Publishing Associates et Wiley-Interscience, New York.
12. Sokoloff P., Riou J.F., Martres M.P. et Schwartz J.C. "Presence of dopamine D-2 receptors in human tumoral cell lines". Biochem. Biophys. Res. Comm. 1989, 162: 575-582.
13. Sokoloff P., B. Giros, M.P. martres, M.L. Bouthenet et J.C. Schwartz. "Molecular cloning and Characterization of a novel dopamine receptor (D₃) as a target for neuroleptics". Nature, 1990, 347, p. 146 à 151.

## Revendications

1. Polypeptide ayant une activité de récepteur dopaminergique contenant :
. la séquence de 400 acides aminés de la Figure 1,
. ou un fragment de cette séquence, ce fragment étant tel que
* soit il contient néanmoins les sites contenus dans cette séquence et dont la présence est nécessaire pour que, lorsque ce fragment est exposé à la surface d'une cellule, il soit capable de lier la dopamine, ses agonistes ou antagonistes de manière spécifique et mesurable, par exemple au moyen d'un ligand radioactif selon la technique décrite dans Sokoloff et al. (1980)(6) et Martres et al. (1985)(7), ces sites étant différentsdes sites contenus dans le récepteur D-3 de rat,
* soit il est susceptible d'être reconnu par des anticorps qui reconnaissent également la susdite séquence de 400 acides aminés, mais ne reconnaissent ni le récepteur dopaminergique D-1, ni le récepteur dopaminergique D-2, ni le récepteur dopaminergique D-3 de rat,
* soit il est susceptible de générer des anticorps reconnaissant la susdite séquence de 400 acides aminés mais ne reconnaissant ni le récepteur dopaminergique D-1, ni le récepteur dopaminergique D-2, ni le récepteur dopaminergique D-3 de rat, ou polypeptides variants qui correspondent aux polypeptides sus-définis, comportant certaines mutations localisées, sans que les polypeptides ne perdent les propriétés de récepteur dopaminergique, et, en particulier, les polypeptides qui ont l'une au moins des quatre mutations suivantes (les positions des acides aminés susceptibles d'être mutés étant repérées par rapport à la Figure 1) :
Ser en position 9 est remplacé par Gly,
Ala en position 79 est remplacé par Val,
Val en position 189 est remplacé par Ile,
Ile en position 397 est remplacé par Thr,
et notamment des polypeptides qui présentent les quatre mutations indiquées ci-dessus.

2. Polypeptide selon la revendication 1, caractérisé en ce qu'il est constitué par la séquence, représentée sur la Figure 1, s'étendant de l'extrémité constituée par l'acide aminé en position 1 à celle constituée par l'extrémité en position 400.

3. Acide nucléique caractérisé en ce qu'il comprend ou est constitué par un enchaînement de nucléotides qui, à l'issue de la traduction ou à l'issue de la transcription et de la traduction conduit à l'un quelconque des polypeptides selon l'une des revendications 1 ou 2.

4. Acide nucléique selon la revendication 3, caractérisé en ce qu'il comprend ou est constitué par - l'enchaînement de nucléotides représenté sur la Figure 3, s'étendant de l'extrémité constituée par le nucléotide 1 à celle constituée par le nucléotide 574, ou par
- l'enchaînement de nucléotides représenté sur la Figure 4, s'étendant de l'extrémité constituée par le nucléotide 1 à celle constituée par le nucléotide 306,
- l'enchaînement de nucléotides représenté sur la Figure 1, s'étendant de l'extrémité constituée par le nucléotide -6 à celle constituée par le nucléotide 1266,
ou
- par l'enchaînement de nucléotides représenté sur la Figure 1, s'étendant de l'extrémité constituée par le nucléotide 1 à celle constituée par le nucléotide 1200 ou les acides nucléiques possédant l'une au moins des six mutations suivantes (les positions des nucléotides susceptibles d'être mutés étant repérées par rapport à la Figure 1) :
A en position 25 est remplacé par G,
A en position 51 est remplacé par G,
C en position 236 est remplacé par T,
G en position 565 est remplacé par A,
T en position 876 est remplacé par C,
T en position 1190 est remplacé par C,
et, en particulier, les acides nucléiques comportant l'ensemble des six mutations.

5. Vecteur recombinant, en particulier pour le clonage et/ou l'expression, notamment du type plasmide, cosmide, phage ou virus, caractérisé en ce qu'il contient un acide nucléique selon l'une des revendications 3 et 4 en l'un de ses sites non essentiels pour sa réplication.

6. Vecteur recombinant selon la revendication 5, caractérisé en ce qu'il contient en l'un de ses sites non essentiels pour sa réplication des éléments nécessaires pour promouvoir l'expression d'une séquences d'acides aminés selon les revendications 1 ou 2, dans un hôte cellulaire et éventuellement un promoteur reconnu par les polymérases de l'hôte cellulaire, en particulier un promoteur inductible et éventuellement une séquence signal et une séquence d'ancrage.

7. Hôte cellulaire transformé par un vecteur recombinant selon l'une quelconque des revendications 5 ou 6 et comprenant les éléments de régulation permettant l'expression de la séquence nucléotidique codant pour le polypeptide selon l'une des revendications 1 ou 2 dans cet hôte.

8. Hôte cellulaire transformé selon la revendication 7, caractérisé en ce qu'il est choisi parmi les bactéries, notamment E. coli.

9. Hôte cellulaire transformé selon la revendication 7, caractérisé en ce qu'il est choisi parmi les organismes eucaryotes tels que des cellules CHO ou COS-7.

10. Anticorps caractérisé(s) en ce qu'il(s) est (ou sont) dirigé(s) de façon spécifique contre un polypeptide selon l'une quelconque des revendications 1 ou 2, et en ce qu'il(s) ne reconnaît (ou reconnaissent) ni le récepteur dopaminergique D-1, ni le récepteur dopaminergique D-2, et en particulier celui ou ceux reconnaissant les séquences d'acides aminés suivantes:
- celle définie par la séquence d'acides aminés représentée sur la Figure 1 s'étendant de l'extrémité constituée par l'acide aminé en position 243 à celle constituée par l'acide aminé en position 277.

11. Sonde nucléotidique caractérisée en ce qu'elle s'hybride avec l'un des acides nucléiques selon les revendications 3 et 4 ou leur séquence complémentaire dans les conditions d'hybridation telles qu'elle ne s'hybride pas avec les gènes ou ARN messager des récepteurs dopaminergiques D-1 ou D-2 et du récepteur dopaminergique D-3 de rat, et en particulier les sondes choisies parmi les sondes nucléotidiques suivantes :
. - celle définie par la séquence d'acides nucléiques représentée sur la Figure 1, s'étendant de l'extrémité constituée par le nucléotide en position -6, à celle constituée par le nucléotide en position 1266,
. - celle définie par la séquence d'acides nucléiques représentée sur la Figure 1, s'étendant de l'extrémité constituée par le nucléotide en position 1 à celle constituée par le nucléotide en position 1200,
. - celle définie par la séquence d'acides nucléiques représentée sur la Figure 1, s'étendant de l'extrémité constituée par le nucléotide en position 720, à celle constituée par le nucléotide en position 831,
. - celle définie par la séquence d'acides nucléiques représentée sur la Figure 3, s'étendant de l'extrémité constituée par le nucléotide en position 1, à celle constituée par le nucléotide en position 574,
. - celle définie par la séquence d'acides nucléiques représentée sur la Figure 4, s'étendant de l'extrémité constituée par le nucléotide en position 1, à celle constituée par le nucléotide en position 306, ou leur séquence nucléotidique complémentaire.

12. Procédé de détection de la capacité d'une molécule à se comporter comme ligand vis-à-vis d'un polypeptide selon l'une quelconque des revendications 1 ou 2, caractérisé par :
- la mise en contact de la molécule avec un hôte cellulaire préalablement transformé par un vecteur lui-même modifié par un insérat codant pour le susdit polypeptide, cet hôte portant à sa surface un ou plusieurs sites spécifiques de ce polypeptide, le cas échéant après induction de l'expression de cet insérat, cette mise en contact étant effectuée dans des conditions permettant la formation d'une liaison entre l'un au moins de ces sites spécifiques et ladite molécule dès lors qu'elle s'avérerait effectivement posséder une affinité pour ce polypeptide,
- la détection de la formation éventuelle d'un complexe du type ligand-polypeptide.

13. Procédé pour l'étude de l'affinité d'un polypeptide selon l'une quelconque des revendications 1 ou 2, pour un ou plusieurs ligands déterminés, caractérisé par :
- la transformation d'un hôte cellulaire compétent avec un vecteur, notamment un plasmide ou un phage, dans lequel avait auparavant été insérée une séquence de nucléotides codant pour le récepteur D-3 humain (insérat), sous le contrôle d'éléments de régulation, notamment d'un promoteur reconnu par les polymérases de l'hôte cellulaire et permettant l'expression dans l'hôte cellulaire utilisé de ladite séquence de nucléotides,
- la culture de l'hôte cellulaire transformé dans des conditions permettant l'expression dudit insérat, et le transport du récepteur D-3 humain exprimé vers la membrane de façon telle que les séquences transmembranaires de récepteur D-3 soient exposées à la surface de l'hôte cellulaire transformé,
- la mise en contact de cet hôte cellulaire avec ces ligands déterminés,
- la détection d'une réaction affine entre ledit hôte cellulaire transformé et lesdits ligands déterminés.

14. Procédé de diagnostic in vitro de l'expression pathologique du récepteur dopaminergique D-3 humain, dans lequel on utilise l'une ou plusieurs quelconques des sondes définies selon la revendication 11, comprenant les étapes suivantes :
- l'amplification préalable possible des quantités de séquence nucléotidique selon l'une quelconque des revendications 3 ou 4 susceptible d'être contenue dans un échantillon biologique prélevé sur un patient, au moyen d'amorces d'ADN,
- la mise en contact de l'échantillon biologique indiqué ci-dessus avec une sonde nucléotidique selon la revendication 11,
dans des conditions permettant la production d'un complexe d'hybridation formé de ladite sonde et ladite séquence nucléotidique,
- la détection du complexe d'hybridation ci-dessus qui a pu se former.

15. Procédé de diagnostic in vitro d'anomalies génétiques, notamment polymorphismes du gène codant pour le récepteur dopaminergique D-3 humain, susceptibles d'être corrélées à des affections psychiatriques, neurologiques, cardio-vasculaires ou neuroendocriniennes, à partir d'un échantillon biologique, tel que le sang, prélevé chez un individu, selon un procédé comprenant les étapes suivants :
- le traitement de l'acide nucléique issu de l'échantillon biologique sus-mentionné réalisé à l'aide d'une enzyme de restriction dans des conditions permettant l'obtention de fragments de restriction issus du clivage dudit acide nucléique au niveau de ces sites de restriction reconnus par ladite enzyme,
- la mise en contact d'une sonde nucléotidique selon la revendication 11, susceptible de s'hybrider avec les fragments sus-mentionnés dans des conditions permettant la production éventuelle de complexes d'hybridation entre ladite sonde et les fragments de restriction sus-mentionnés,
- la détection des susdits complexes d'hybridation,
- la mesure de taille des éventuels fragments de restriction polymorphes engagés dans les complexes d'hybridation sus-mentionnés.

16. Procédé de diagnostic in vitro de détection de mutations ponctuelles de l'acide nucléique ou d'un fragment de l'acide nucléique codant pour le récepteur dopaminergique D-3 humain selon les revendications 3 ou 4, chez un individu, comprenant les étapes suivantes :
- la mise en contact d'une sonde nucléotidique selon la revendication 11, avec un prélèvement biologique, par exemple du sang, dans des conditions permettant le production éventuelle d'un complexe d'hybridation formé entre la sonde et l'acide nucléique ou le fragment d'acide nucléique sus-mentionné,
- la détection du susdit complexe d'hybridation,
- le séquençage de l'acide nucléique ou du fragment d'acide nucléique intervenant dans le susdit complexe d'hybridation,
- la comparaison de la séquence de l'acide nucléique ou du fragment d'acide nucléique intervenant dans le susdit complexe d'hybridation avec la séquence de l'acide nucléique (ne présentant pas de mutation) ou du fragment de l'acide nucléique (ne présentant pas de mutation) du récepteur dopaminergique D-3 humain.

17. Procédé de diagnostic in vitro à partir du prélèvement biologique de l'expression pathologique du récepteur dopaminergique D-3 humain, dans lequel on utilise l'un ou plusieurs anticorps selon la revendication 10 comprenant les étapes suivantes :
- la mise en contact d'un anticorps selon la revendication 10 avec le susdit prélèvement biologique dans des conditions permettant la production éventuelle d'un complexe immunologique formé entre le récepteur dopaminergique D-3 humain ou un dérivé de celui-ci comportant une ou plusieurs mutations localisées et conservant les propriétés de récepteur D-3 dopaminergique humain d'une part, et l'anticorps de l'invention d'autre part, et,
- la détection du susdit complexe immunologique formé.

18. Procédé d'évaluation du pouvoir agoniste ou antagoniste vis à vis du récepteur dopaminergique D-3 humain d'une molécule ou médicament dans lequel on utilise une cellule hôte définie selon les revendications 7 et 9, transformée avec un vecteur contenant le gène de la sous-unité α de la protéine transductrice G₀,
- s'il y a augmentation de la concentration de calcium intracellulaire par rapport à la concentration de calcium intracellulaire en l'absence de ladite molécule, on en déduit que la molécule est un agoniste vis à vis de D-3;
- s'il n'y a pas modification de la concentration de calcium intracellulaire, ou s'il y a diminution de la concentration de calcium intracellulaire, par rapport à la concentration de calcium intracellulaire en l'absence de ladite molécule, on met la susdite cellule hôte en présence de dopamine et de la susdite molécule, et s'il y a diminution de la concentration de calcium intracellulaire par rapport à celle obtenue en présence de dopamine, on en déduit que la molécule est un antagoniste vis-à-vis de D-3.

## Claims

1. Polypeptide having a dopaminergic receptor activity containing:
. the sequence of 400 amino acids shown in Figure 1,
. or a fragment of this sequence, this fragment being such that
* either it contains nonetheless the sites contained in this sequence and the presence of which is necessary in order that, when this fragment is exposed at the surface of a cell, it is capable of binding dopamine, its agonists or antagonists in a specific and measurable manner, for example by means of a radioactive ligand according to the procedure described in Sokoloff et al. (1980)(6) and Martres et al. (1985)(7), these sites being different from the sites contained in the D-3 receptor of the rat,
* or it is capable of being recognized by antibodies which also recognize the above-mentioned sequence of 400 amino acids but which do not recognize either the D-1 dopaminergic receptor, or the D-2 dopaminergic receptor or the D-3 dopaminergic receptor of the rat,
* or it is capable of generating antibodies which recognize the above-mentioned sequence of 400 amino acids but which do not recognize either the D-1 dopaminergic receptor, or the D-2 dopaminergic receptor or the D-3 dopaminergic receptor of the rat, or variant polypeptides which correspond to the polypeptides defined above bearing certain localized mutations without the polypeptides losing the properties of a dopaminergic receptor and, in particular, the polypeptides which have at least one of the following four mutations (the positions of the amino acids likely to be mutated being localized with reference to Figure 1):
Ser at position 9 is replaced by Gly,
Ala at position 79 is replaced by Val,
Val at position 189 is replaced by Ile,
Ile at position 397 is replaced by Thr,
and, in particular, polypeptides which exhibit the four mutations indicated above.

2. Polypeptide according to Claim 1, characterized in that it is constituted by the sequence shown in Figure 1, extending from the extremity constituted by the amino acid at position 1 to that constituted by the amino acid at position 400.

3. Nucleic acid characterized in that it comprises or is constituted by a chain of nucleotides which, subsequent to translation or after transcription and translation lead to any one of the polypeptides according to one of the Claims 1 or 2.

4. Nucleic acid according to Claim 3, characterized in that it comprises or is constituted by the chain of nucleotides shown in Figure 3, extending from the end constituted by the nucleotide 1 to that constituted by the nucleotide 574, or by
- the chain of nucleotides shown in Figure 4 extending from the end constituted by the nucleotide 1 to that constituted by the nucleotide 306,
- the chain of nucleotides shown in Figure 1, extending from the end constituted by the nucleotide -6 to that constituted by the nucleotide 1266,
or
- by the chain of nucleotides shown in Figure 1, extending from the end constituted by the nucleotide 1 to that constituted by the nucleotide 1200 or the nucleic acids possessing at least one of the following six mutations (the positions of the nucleotides likely to be mutated being located with reference to Figure 1):
A at position 25 is replaced by G,
A at position 51 is replaced by G,
C at position 236 is replaced by T,
G at position 565 is replaced by A,
T at position 876 is replaced by C,
T at position 1190 is replaced by C,
and, in particular, the nucleic acids containing all of the six mutations.

5. Recombinant vector, in particular for cloning and/or expression, in particular of the plasmid, cosmid, phage or virus type, characterized in that it contains a nucleic acid according to one of the Claims 3 and 4 at one of its sites inessential for its replication.

6. Recombinant vector according to Claim 5, characterized in that it contains at one of its sites inessential for its replication elements necessary to promote the expression of a sequence of amino acids according to Claims 1 or 2 in a cell host and, where necessary, a promoter recognized by the polymerases of the cell host, in particular an inducible promoter and possibly a signal sequence and an anchoring sequence.

7. Cell host transformed by a recombinant vector according to any one of the Claims 5 or 6 and comprising regulatory elements making possible the expression of the nucleotide sequence coding for the polypeptide according to one of the Claim 1 or 2 in this host.

8. Cell host transformed according to Claim 7, characterized in that it is selected from among the bacteria, in particular E.coli.

9. Cell host transformed according to Claim 7, characterized in that it is selected from among eukaryotic organisms such as CHO or COS-7 cells.

10. Antibody/antibodies characterized in that it/they is/are directed specifically against a polypeptide according to any one of the Claims 1 or 2, and in that it/they does/do not recognize either the D-1 dopaminergic receptor or the D-2 dopaminergic receptor, and in particular it or they does/do recognize the following amino acid sequences:
- that defined by the amino acid sequence shown in Figure 1 extending from the extremity constituted by the amino acid at position 243 to that constituted by the amino acid at position 277.

11. Nucleotide probe characterized in that it hybridizes with one of the nucleic acids according to Claims 3 and 4 or their complementary sequence under conditions of hybridization such that it does not hybridize with the genes or messenger RNA of the D-1 or D-2 dopaminergic receptors and the D-3 dopaminergic receptor of the rat, and in particular the probes selected from among the following nucleotide probes:
. - that defined by the sequence of nucleotides shown in Figure 1, extending from the end constituted by the nucleotide at position -6 to that constituted by the nucleotide at position 1266,
. - that defined by the nucleotide sequence shown in Figure 1 extending from the end constituted by the nucleotide at position 1 to that constituted by the nucleotide at position 1200,
. - that defined by the nucleotide sequence shown in Figure 1 extending from the end constituted by the nucleotide at position 720 to that constituted by the nucleotide at position 831,
. - that defined by the nucleotide sequence shown in Figure 3 extending from the end constituted by the nucleotide at position 1 to that constituted by the-nucleotide at position 574,
. - that defined by the nucleotide sequence shown in Figure 4 extending from the end constituted by the nucleotide at position 1 to that constituted by the nucleotide at position 306, or their complementary nucleotide sequence.

12. Procedure for the detection of the capacity of a molecule to behave as a ligand towards a polypeptide according to any one of the Claims 1 or 2, characterized by :
- the placing in contact of the molecule with a cell host transformed beforehand by a vector, itself modified by an insert coding for the above-mentioned polypeptide, this host bearing at its surface one or several sites specific for this polypeptide, where appropriate after induction of the expression of this insert, this placing in contact being performed under conditions leading to the formation of a bond between at least one of these specific sites and the said molecule provided that it has been proved effectively to possess an affinity for this polypeptide,
- the detection of the possible formation of a complex of the ligand-polypeptide type.

13. Procedure for the study of the affinity of a polypeptide according to any one of the Claims 1 or 2, for one or several specific ligands, characterized by:
- the transformation of a competent cell host with a vector, in particular a plasmid or a phage, in which a sequence of nucleotides coding for the human D-3 receptor (insert) had previously been inserted,
under the control of regulatory elements, in particular of a promoter recognized by the polymerases of the cell host and which make possible the expression of the said nucleotide sequence in the cell host used,
- the culture of the transformed cell host under conditions permitting the expression of the said insert, and the transport of the human D-3 receptor expressed towards the membrane in a manner such that the transmembrane sequences of the D-3 receptor are exposed at the surface of the transformed cell host,
- the placing in contact of this cell host with these specific ligands,
- the detection of an affinity reaction between the said transformed cell host and the said specific ligands.

14. Procedure for the in vitro diagnosis of the pathological expression of the human D-3 dopaminergic receptor, in which one or more of any of the probes defined according to Claim 11 is/are used, comprising the following steps:
- the possible prior amplification of the amounts of nucleotide sequence according to any one of the Claims 3 or 4, likely to be contained in a biological sample taken from a patient,by means of DNA primers,
- the placing in contact of the biological sample indicated above with a nucleotide probe according to Claim 11,
under conditions leading to the production of a hybridization complex formed between the said probe and the said nucleotide sequence,
- the detection of the above hybridization complex which has been able to form.

15. Procedure for the in vitro diagnosis of genetic anomalies, in particular polymorphisms of the gene coding for the human D-3 dopaminergic receptor, susceptible of being correlated with psychiatric, neurological, cardio-vascular or neuroendocrine diseases, starting from a biological sample, such as blood, taken from an individual, according to a procedure comprising the following steps:
- the treatment of the nucleic acid derived from the above-mentioned biological sample with the aid of a restriction enzyme leading to the production of restriction fragments derived from the cleavage of the said nucleic acid at those restriction sites recognized by the said enzyme,
- the placing in contact of a nucleotide probe according to Claim 11, capable of hybridizing with the above-mentioned fragments under conditions leading to the possible production of hybridization complexes between the said probe and the above-mentioned restriction fragments,
- the detection of the above-mentioned hybridization complexes,
- the measurement of the size of the polymorphic restriction fragments possibly incorporated into the above-mentioned hybridization complexes.

16. Procedure for the in vitro diagnosis of point mutations in the nucleic acid or in a fragment of the nucleic acid coding for the human D-3 dopaminergic receptor according to Claims 3 or 4 in an individual, comprising the following steps:
- the placing in contact of a nucleotide probe according to Claim 11 with a biological sample, for example blood, under conditions leading to the possible production of a hybridization complex formed between the probe and the above-mentioned nucleic acid or nucleic acid fragment,
- the detection of the above-mentioned hybridization complex,
- the sequencing of the nucleic acid or the nucleic acid fragment implicated in the above-mentioned hybridization complex,
- the comparison of the sequence of the nucleic acid or nucleic acid fragment implicated in the above-mentioned hybridization complex with the sequence of the nucleic acid (not exhibiting a mutation) or of the nucleic acid fragment (not exhibiting a mutation) of the human D-3 dopaminergic receptor.

17. Procedure for the in vitro diagnosis of the pathological expression of the human D-3 dopaminergic receptor in a biological sample, in which one or more antibodies according to Claim 10 are used, which comprises the following steps:
- the placing in contact of an antibody according to Claim 10 with the above-mentioned biological sample under conditions leading to the possible production of an immunological complex formed between the human D-3 dopaminergic receptor or a product which is derived from it comprising one or several localized mutations and retaining the properties of human dopaminergic D3 receptor on the one hand, and the antibody of the invention on the other hand, and,
- the detection of the above-mentioned immunological complex formed.

18. Procedure for the evaluation of the agonist or antagonist potency towards the human D-3 dopaminergic receptor of a molecule or medicine in which is used a cell host defined according to claims 7 and 9, transformed by a vector containing the gene for the α subunit of the G₀ transducing protein,
- if there is an increase in the intracellular calcium concentration compared with the intracellular calcium concentration in the absence of the said molecule, it can be deduced that the molecule is a D-3 agonist;
- if there is no change in the intracellular calcium concentration, or if there is a decrease in the intracellular calcium concentration compared with the intracellular calcium concentration in the absence of the said molecule, the above-mentiohed cell host is placed in the presence of dopamine and the above-mentioned molecule and if there is a decrease in the intracellular calcium concentration compared with that produced in the presence of dopamine, it can be deduced that the molecule is a D-3 antagonist.

## Patentansprüche

1. Polypeptid mit einer Aktivität eines dopaminergen Rezeptors, umfassend:
- die Sequenz von 400 Aminosäuren der Figur 1,
- oder ein Fragment dieser Sequenz, wobei dieses Fragment dergestalt ist, daß
* es entweder gleichwohl die in dieser Sequenz enthaltenen Stellen enthält, deren Anwesenheit erforderlich ist, damit, wenn dieses Fragment auf der Oberfläche einer Zelle exponiert wird, es in der Lage ist, Dopamin, seine Agonisten.oder Antagonisten auf spezifische und meßbare Weise, beispielsweise mittels eines radioaktiven Liganden gemäß der in Sokoloff et al. (1980)(6) und Martres et al. (1985)(7) beschriebenen Technik, zu binden, wobei diese Stellen sich von den in dem Rezeptor D-3 der Ratte enthaltenen Stellen unterscheiden,
* oder es durch Antikörper erkannt werden kann, die gleichfalls die Sequenz von 400 Aminosäuren erkennen, aber weder den dopaminergen Rezeptor D-1, noch den dopaminergen Rezeptor D-2, noch den dopaminergen Rezeptor D-3 der Ratte erkennen,
* oder es Antikörper erzeugen kann, die die Sequenz von 400 Aminosäuren erkennen, aber weder den dopaminergen Rezeptor D-1, noch den dopaminergen Rezeptor D-2, noch den dopaminergen Rezeptor D-3 der Ratte erkennen, oder
Polypeptidvarianten, die den vorstehend definierten Polypeptiden entsprechen und bestimmte lokalisierte Mutationen tragen, ohne daß die Polypeptide die Eigenschaften eines dopaminergen Rezeptors verlieren, und insbesondere die Polypeptide, die mindestens eine der vier folgenden Mutationen aufweisen (wobei die Positionen der Aminosäuren, die mutiert werden können, bezogen auf die Figur 1 gekennzeichnet sind):
Ser an Position 9 wird durch Gly ersetzt,
Ala an Position 79 wird durch Val ersetzt,
Val an Position 189 wird durch Ile ersetzt,
Ile an Position 397 wird durch Thr ersetzt,
und insbesondere die Polypeptide, die die vier vorstehend angegebenen Mutationen aufweisen.

2. Polypeptid nach Anspruch 1, dadurch gekennzeichnet, daß es durch die in der Figur 1 angegebene Sequenz gebildet wird, die sich von dem durch die Aminosäure an Position 1 gebildeten Ende bis zu jenem, das durch das Ende an Position 400 gebildet wird, erstreckt.

3. Nukleinsäure, dadurch gekennzeichnet, daß sie umfaßt oder gebildet wird durch eine Aneinanderreihung von Nukleotiden, die am Ende der Translation oder am Ende der Transkription und der Translation zu einem der Polypeptide nach einem der Ansprüche 1 oder 2 führt.

4. Nukleinsäure nach Anspruch 3, dadurch gekennzeichnet, daß sie umfaßt oder gebildet wird durch
- die Aneinanderreihung von Nukleotiden, die in Figur 3 angegeben ist und sich von dem durch das Nukleotid 1 gebildeten Ende bis zu jenem, das durch das Nukleotid 574 gebildet wird, erstreckt, oder durch
- die Aneinanderreihung von Nukleotiden, die in Figur 4 angegeben ist und sich von dem durch das Nukleotid 1 gebildeten Ende bis zu jenem, das durch das Nukleotid 306 gebildet wird, erstreckt,
- die Aneinanderreihung von Nukleotiden, die in Figur 1 angegeben ist und sich von dem durch das Nukleotid -6 gebildeten Ende bis zu jenem, das durch das Nukleotid 1266 gebildet wird, erstreckt, oder
- durch die Aneinanderreihung von Nukleotiden, die in Figur 1 angegeben ist und sich von dem durch das Nukleotid 1 gebildeten Ende bis zu jenem, das durch das Nukleotid 1200 gebildet wird, erstreckt,
oder die Nukleinsäuren, die mindestens eine der folgenden sechs Mutationen aufweisen (wobei die Positionen der Nukleotide, die mutiert sein können, bezogen auf die Figur 1 gekennzeichnet sind):
A an Position 25 wird durch G ersetzt,
A an Position 51 wird durch G ersetzt,
C an Position 236 wird durch T ersetzt,
G an Position 565 wird durch A ersetzt,
T an Position 876 wird durch C ersetzt,
T an Position 1190 wird durch C ersetzt,
und insbesondere die Nukleinsäuren, die die Gesamtheit der sechs Mutationen umfassen.

5. Rekombinanter Vektor, insbesondere für die Klonierung und/oder die Expression, insbesondere vom Typ Plasmid, Cosmid, Phage oder Virus, dadurch gekennzeichnet, daß er eine Nukleinsäure nach einem der Ansprüche 3 und 4 in einer seiner für seine Replikation nicht essenziellen Stellen enthält.

6. Rekombinanter Vektor nach Anspruch 5, dadurch gekennzeichnet, daß er in einer seiner für seine Replikation nicht essenziellen Stellen Elemente, die zur Förderung der Expression einer Aminosäuresequenz nach den Ansprüchen 1 oder 2 in einem zellulären Wirt erforderlich sind, und gegebenenfalls einen von den Polymerasen des zellulären Wirts erkannten Promotor, insbesondere eine induzierbaren Promotor, und gegebenenfalls eine Signalsequenz und eine Ankersequenz enthält.

7. Durch einen rekombinanten Vektor nach einem der Ansprüche 5 oder 6 transformierter zellulärer Wirt, der die Regulationselemente umfaßt, die die Expression der Nukleotidsequenz, die das Polypeptid nach einem der Ansprüche 1 oder 2 kodiert, in diesem Wirt ermöglichen.

8. Transformierter zellulärer Wirt nach Anspruch 7, dadurch gekennzeichnet, daß er unter den Bakterien, insbesondere E. coli, ausgewählt wird.

9. Transformierter zellulärer Wirt nach Anspruch 7, dadurch gekennzeichnet, daß er unter den eukaryotischen Organismen, wie den CHO- oder COS-7-Zellen ausgewählt wird.

10. Antikörper, dadurch gekennzeichnet, daß er (sie) auf spezifische Weise gegen ein Polypeptid nach einem der Ansprüche 1 oder 2 gerichtet ist (oder sind) und daß er (sie) weder den dopaminergen Rezeptor D-1, noch den dopaminergen Rezeptor D-2 erkennt (oder erkennen), und insbesondere jener oder jene, der bzw. die die folgenden Aminosäuresequenzen erkennt bzw. erkennen:
- jene, die durch die in Figur 1 angegebene Aminosäuresequenz, die sich von dem durch die Aminosäure an Position 243 gebildeten Ende bis zu jenem, das durch die Aminosäure an Position-277 gebildet wird, erstreckt, definiert wird.

11. Nukleotidsonde, dadurch gekennzeichnet, daß sie mit einer der Nukleinsäuren nach den Ansprüchen 3 und 4 oder deren komplementären Sequenzen.unter solchen Hybridisierungsbedingungen, unter denen sie mit den Genen oder der mRNA der dopaminergen Rezeptoren D-1 oder D-2 und des dopaminergen Rezeptors D-3 der Ratte nicht hybridisiert, hybridisiert, und insbesondere die Sonden, die aus den folgenden Nukleotidsonden ausgewählt werden:
. - jene, die durch die in Figur 1 angegebene Nukleinsäuresequenz, die sich von dem durch das Nukleotid an Position -6 gebildeten Ende bis zu jenem, das durch das Nukleotid an Position 1266 gebildet wird, erstreckt, definiert wird,
. - jene, die durch die in Figur 1 angegebene Nukleinsäuresequenz, die sich von dem durch das Nukleotid an Position 1 gebildeten Ende bis zu jenem, das durch das Nukleotid an Position 1200 gebildet wird, erstreckt, definiert wird,
. - jene, die durch die in Figur 1 angegebene Nukleinsäuresequenz, die sich von dem durch das Nukleotid an Position 720 gebildeten Ende bis zu jenem, das durch das Nukleotid an Position 831 gebildet wird, erstreckt, definiert wird,
. - jene, die durch die in Figur 3 angegebene Nukleinsäuresequenz, die sich von dem durch das Nukleotid an Position 1 gebildeten Ende bis zu jenem, das durch das Nukleotid an Position 574 gebildet wird, erstreckt, definiert wird,
. - jene, die durch die in Figur 4 angegebene Nukleinsäuresequenz, die sich von dem durch das Nukleotid an Position 1 gebildeten Ende bis zu jenem, das durch das Nukleotid an Position 306 gebildet wird, erstreckt, definiert wird,
oder deren komplementäre Nukleotidsequenz.

12. Verfahren zum Nachweis der Fähigkeit eines Moleküls, sich als Ligand gegenüber einem Polypeptid gemäß einem der Ansprüche 1 oder 2 zu verhalten, gekennzeichnet durch:
- das Inkontaktbringen des Moleküls mit einem zelluären Wirt, der vorab transformiert worden ist durch einen Vektor, der seinerseits durch ein Insert, das das Polypeptid kodiert, modifiziert worden ist, wobei dieser Wirt auf seiner Oberfläche eine oder mehrere spezifische Stellen dieses Polypeptids trägt, gegebenenfalls nach Induktion der Expression dieses Inserts, wobei dieses Inkontaktbringen unter Bedingungen ausgeführt wird, die die Bildung einer Bindung zwischen mindestens einer dieser spezifischen Stellen und dem Molekül ermöglichen, sobald es sich herausstellt, daß es tatsächlich eine Affinität für dieses Polypeptid aufweist,
- den Nachweis der gegebenenfalls erfolgten Bildung eines Komplexes des Typs Ligand-Polypeptid.

13. Verfahren zur Untersuchung der Affinität eines Polypeptids nach einem der Ansprüche 1 oder 2 für einen oder mehrere bestimmte Liganden, gekennzeichnet durch:
- die Transformation eines kompetenten zellulären Wirts mit einem Vektor, insbesondere einem Plasmid oder einem Phagen, in den man vorab eine Nukleotidsequenz, die den humanen Rezeptor D-3 kodiert, (Insert) unter der Kontrolle von Regulationselementen, insbesondere eines Promotors, der von den Polymerasen des zellulären Wirts erkannt wird und die Expression der Nukleotidsequenz in dem verwendeten zellulären Wirt ermöglicht, insertiert hat,
- die Züchtung des transformierten zellulären Wirts unter Bedingungen, die die Expression des Inserts und den Transport des exprimierten humanen Rezeptors D-3 zu der Membran dergestalt ermöglichen, daß die Transmembransequenzen des Rezeptors D-3 auf der Oberfläche des transformierten zellulären Wirts exponiert werden,
- das Inkontaktbringen dieses zellulären Wirts mit diesen bestimmten Liganden,
- den Nachweis einer Affinitätsreaktion zwischen dem transformierten zellulären Wirt und denbestimmten Liganden.

14. in vitro-Diagnoseverfahren für die pathologische Expression des humanen dopaminergen Rezeptors D-3, in welchem man eine oder beliebige mehrere der gemäß Anspruch 11 definierten Sonden verwendet, welches die folgenden Schritte umfaßt:
- die mögliche, vorab erfolgende Amplifizier'ung der Mengen an Nukleotidsequenz nach einem der Ansprüche 3 oder 4, die in einer biologischen Probe, die einem Patienten abgenommen worden ist, enthalten sein könnte, mittels DNA-Primer,
- das Inkontaktbringen der vorstehend angegebenen biologischen Probe mit einer Nukleotidsonde nach Anspruch 11 unter Bedingungen, die die Bildung eines Hybridisierungskomplexes, der aus der Sonde und der Nukleotidsequenz gebildet wird, ermöglichen,
- den Nachweis des Hybridisierungskomplexes, der sich möglicherweise gebildet hat.

15. in vitro-Diagnoseverfahren für genetische Anomalien, insbesondere Polymorphismen des den humanen dopaminergen Rezeptor D-3 kodierenden Gens, die mit psychiatrischen, neurologischen, kardiovaskulären oder neuroendokrinen Erkrankungen korreliert werden können, ausgehend von einer biologischen Probe, wie Blut, das einem Patienten abgenommen worden ist, gemäß einem Verfahren, das die folgenden Schritte umfaßt:
- die Behandlung der aus der biologischen Probe stammenden Nukleinsäure, die mittels eines Restriktionsenzyms unter Bedingungen ausgeführt wird, die die Gewinnung von Restriktionsfragmenten ermöglichen, die aus der Spaltung der Nukleinsäure auf der Ebene dieser von dem Enzym erkannten Restriktionsstellen stammen,
- das Inkontaktbringen einer Nukleotidsonde nach Anspruch 11, die zu einer Hybridisierung in der Lage ist, mit den Fragmenten unter Bedingungen, die die gegebenenfalls erfolgende Bildung von Hybridisierungskomplexen zwischen der Sonde und den Restriktionsfragmenten ermöglichen,
- den Nachweis der Hybridisierungskomplexe,
- das Messen der Größe der gegebenenfalls vorliegenden polymorphen Restriktionsfragmente, die an den Hybridisierungskomplexen beteiligt sind.

16. in vitro-Diagnoseverfahren zum Nachweis von Punktmutationen der Nukleinsäure oder eines Fragments der Nukleinsäure, die den humanen dopaminergen Rezeptor D-3 kodiert, gemäß den Ansprüchen 3 oder 4 bei einem Patienten, welches die folgenden Schritte umfaßt:
- das Inkontaktbringen einer Nukleotidsonde nach Anspruch 11 mit einem biologischen Untersuchungsmaterial, beispielsweise Blut, unter Bedingungen, die die gegebenenfalls erfolgende Bildung eines Hybridisierungskomplexes, der zwischen der Sonde und der Nukleinsäure oder dem Nukleinsäurefragment gebildet wird, ermöglichen,
- den Nachweis des Hybridisierungskomplexes,
- die Sequenzierung der Nukleinsäure oder des Nukleinsäurefragments, die an dem Hybridisierungskomplex beteiligt sind,
- den Vergleich der Sequenz der Nukleinsäure oder des Nukleinsäurefragments, die an dem Hybridisierungskomplex beteiligt sind, mit der Sequenz der Nukleinsäure (die keine Mutation aufweist) oder des Nukleinsäurefragments (das keine Mutation aufweist) des humanen dopaminergen Rezeptors D-3.

17. in vitro-Diagnoseverfahren für die pathologische Expression des humanen dopaminergen Rezeptors D-3 ausgehend von einem biologischen Untersuchungsmaterial, in welchem man einen oder mehrere Antikörper nach Anspruch 10 einsetzt, umfassend die folgenden Schritte:
- das Inkontaktbringen eines Antikörpers nach Anspruch 10 mit dem biologischen Untersuchungsmaterial unter Bedingungen, die die gegebenenfalls erfolgende Bildung eines immunologischen Komplexes ermöglichen, der zwischen dem humanen dopaminergen Rezeptor D-3 oder einem Derivat davon, das eine oder mehrere lokalisierte Mutationen umfaßt und die Eigenschaften des humanen dopaminergen Rezeptors D-3 bewahrt, einerseits und dem Antikörper der Erfindung andererseits gebildet wird, und
- den Nachweis des gebildeten immunologischen Komplexes.

18. Verfahren zur Auswertung der Agonisten- oder Antagonistenfähigkeit eines Moleküls oder Arzneimittels gegenüber dem humanen dopaminergen Rezeptor D-3, bei dem man eine gemäß den Ansprüchen 7 und 9 definierte Wirtszelle einsetzt, die mit einem Vektor, der das Gen der α-Untereinheit des G₀-Transduktionsproteins enthält, transformiert worden ist,
- wenn eine Erhöhung der Konzentration an intrazellulärem Calcium bezogen auf die Konzentration an intrazellulärem Calcium in Abwesenheit des Moleküls auftritt, man daraus schließt, daß das Molekül ein Agonist gegenüber D-3 ist,
- wenn es keine Modifizierung der Konzentration an intrazellulärem Calcium gibt oder wenn eine Verringerung der Konzentration an intrazellulärem Calcium bezogen auf die Konzentration an intrazellulärem Calcium in Abwesenheit des Moleküls auftritt, man die Wirtszelle in Gegenwart von Dopamin und dem genannten Molekül bringt, und wenn eine Verringerung der Konzentration an intrazellulärem Calcium gegenüber jener, die in Gegenwart von Dopamin erhalten wird, auftritt, man daraus schließt, daß das Molekül ein Antagonist gegenüber D-3 ist.
